# EUROPÄISCHE PATENTSCHRIFT

(11) **EP 1 037 671 B1**
(45) Veröffentlichungstag und Bekanntmachung des Hinweises auf die Patenterteilung: **05.02.2003**
(21) Anmeldenummer: 98965773.9
(22) Anmeldetag: 09.12.1998
(51) Int. Cl.: A61K 49/00, A61K 47/48, A61K 49/04, A61P 43/00

(54) **POLYROTAXANE**
POLYROTAXANES
POLYROTAXANES

(30) Priorität: 17.12.1997 DE 19758118
(43) Veröffentlichungstag der Anmeldung: 27.09.2000
(73) Patentinhaber: SCHERING AKTIENGESELLSCHAFT, 13342 Berlin (DE)
(72) Erfinder: PLATZEK, Johannes, D-12621 Berlin (DE); SCHMITT-WILLICH, Heribert, D-12161 Berlin (DE)
(86) Internationale Anmeldenummer: EP9807924
(87) Internationale Veröffentlichungsnummer: WO99030744

(56) Entgegenhaltungen:
- EP-A- 0 766 968
- WO-A-98/01163
- AKIRA HARADA ET AL: "SYNTHESIS OF A TUBULAR POLYMER FROM THREADED CYCLODEXTRINS" NATURE, Bd. 364, Nr. 6437, 5. August 1993, Seiten 516-518, XP000371695
- RAYMO F M ET AL: "POLYROTAXANES AND PSEUDOPOLYROTAXANES" TRENDS IN POLYMER SCIENCE, Bd. 4, Nr. 7, 1. Juli 1996, Seiten 208-211, XP000591869
- CARDENAS D.J. ET AL: "Construction of interlocking and threaded rings using two different transition metals as a templating and connecting centers: Catenanes and rotaxanes incorporating Ru(terpy)2-units in their framework" JOURNAL OF THE AMERICAN CHEMICAL SOCIETY, 1997, 119/11 (2656-2664), XP002054618 USA
- HARADA A. ET AL: "Preparation and Characterization of a polyrotaxane consisting on monodisperse poly(ethylene glycol) and alpha cyclodextrins" J. AM. CHEM. SOC., Bd. 116, 1994, Seiten 3192-3196, XP002096595
- HARADA A ET AL: "PREPARATION AND CHARACTERIZATION OF INCLUSION COMPLEXES OF POLY(PROPYLENE GLYCOL) WITH CYCLODEXTRINS" MACROMOLECULES, Bd. 28, Nr. 24, 20. November 1995, Seiten 8406-8411, XP000539442
- TOPCHIEVA I N ET AL: "SUPERMOLECULAR STRUCTURES BASED ON POLY(ETHYLENE OXIDE)- POLY(PROPYLENE OXIDE) BLOCK COPOLYMERS AND CYCLODEXTRINS" POLYMER SCIENCE, Bd. 36, Nr. 2, 1. Februar 1994, Seiten 221-227, XP000434426
- OOYS T. ET AL: "Synthesis and characterization of biodegradable polyrotaxane as a novel supramolecular-structured drug carrier" J. BIOMATER. SCI. POLYMER EDN, Bd. 8, Nr. 6, 1997, XP002096596

## Beschreibung

Die Erfindung betrifft den in den Patentansprüchen gekennzeichneten Gegenstand, das heißt. neue Polyrotaxane, diese Verbindungen enthaltende Mittel, die Verwendung dieser Verbindungen in der Diagnostik sowie Verfahren zur Herstellung dieser Verbindungen und Mittel.

Polyrotaxane sind Verbindungen, in denen mehrere ringförmige Moleküle auf einem geeigneten Polymerrückgrat aufgefädelt sind. Solche hochmolekularen Molekülgebilde sind u.a. beschrieben von A. Harada et al., J. Am. Chem., Soc. 1994, 116, 3192-96 und G. Wenz et al., Angew. Chem. 104., 201-204 (1992).

Die zur Zeit klinisch eingesetzten Kontrastmittel für die modernen bildgebenden Verfahren Kernspintomographie (MRI) und Computertomographie (CT) [Magnevist ®, Pro Hance ®, Ultravist® und Omniscan ®] verteilen sich im gesamten extrazellulären Raum des Körpers (Intravasalraum und Interstitium). Dieser Verteilungsraum umfaßt etwa 20 % des Körpervolumens.

Extrazelluläre MRI-Kontrastmittel sind klinisch zuerst erfolgreich bei der Diagnostik von zerebralen und spinalen Krankheitsprozessen eingesetzt worden, da sich hier eine ganz besondere Situation hinsichtlich des regionalen Verteilungsraumes ergibt. Im Gehirn und im Rückenmark können extrazelluläre Kontrastmittel im gesunden Gewebe aufgrund der Blut-Hirn-Schranke nicht den Intravasalraum verlassen. Bei krankhaften Prozessen mit Störung der Blut-Hirn-Schranke (z.B. maligne Tumoren, Entzündungen, demyelinisierende Erkrankungen etc.) entstehen innerhalb des Hirns dann Regionen mit erhöhter Blutgefäß-Durchlässigkeit (Permeabilität) für diese extrazellulären Kontrastmittel (Schmiedl et al., MRI of blood-brain barrier permeability in astrocytic gliomas: application of small and large molecular weight contrast media, Magn. Reson. Med. 22: 288, 1991). Durch das Ausnutzen dieser Störung der Gefäßpermeabilität kann erkranktes Gewebe mit hohem Kontrast gegenüber dem gesunden Gewebe erkannt werden.

Außerhalb des Gehirns und des Rückenmarkes gibt es allerdings eine solche Permeabilitätsbarriere für die oben genannten Kontrastmittel nicht (Canty et al., First-pass entry of nonionic contrast agent into the myocardial extravascular space. Effects on radiographic estimate of transit time and blood volume. Circulation 84: 2071, 1991). Damit ist die Anreicherung des Kontrastmittels nicht mehr abhängig von der Gefäßpermeabilität, sondern nur noch von der Größe des extrazellulären Raumes im entsprechenden Gewebe. Eine Abgrenzung der Gefäße gegenüber dem umliegenden interstitiellen Raum bei Anwendung dieser Kontrastmittel ist nicht möglich.

Besonders für die Darstellung von Gefäßen wäre ein Kontrastmittel wünschenswert, das sich ausschließlich im vasalen Raum (Gefäßraum) verteilt. Ein solches blood-pool-agent soll es ermöglichen, mit Hilfe der Kernspintomographie gut durchblutetes von schlecht durchblutetem Gewebe abzugrenzen und somit eine Ischämie zu diagnostizieren. Auch infarziertes Gewebe ließe sich aufgrund seiner Anämie vom umliegenden gesunden oder ischämischen Gewebe abgrenzen, wenn ein vasales Kontrastmittel angewandt wird. Dies ist von besonderer Bedeutung, wenn es z.B. darum geht, einen Herzinfarkt von einer Ischämie zu unterscheiden.

Bisher müssen sich die meisten der Patienten, bei denen Verdacht auf eine kardiovaskuläre Erkrankung besteht (diese Erkrankung ist die häufigste Todesursache in den westlichen Industrieländern), invasiven diagnostischen Untersuchungen unterziehen.
Es besteht daher ein Bedarf an NMR- und Röntgenkontrastmitteln, die den vasalen Raum markieren können (blood-pool-agent). Diese Verbindungen sollen sich durch eine gute Verträglichkeit und durch eine hohe Wirksamkeit (hohe Steigerung der Signalintensität bei MRI) auszeichnen.

Der Ansatz, zumindest einen Teil dieser Probleme durch Verwendung von Komplexen, die an Makro- oder Biomoleküle gebunden sind, zu lösen, war bisher nur sehr begrenzt erfolgreich.

So ist beispielsweise die Anzahl paramagnetischer Zentren in den Komplexen, die in den Europäischen Patentanmeldungen Nr. 0 088 695 und Nr. 0 150 844 beschrieben sind, für eine zufriedenstellende Bildgebung nicht ausreichend.

Erhöht man die Anzahl der benötigten Metallionen durch mehrfache Einführung komplexierender Einheiten in ein makromolekulares Biomolekül, so ist das mit einer nicht tolerierbaren Beeinträchtigung der Affinität und/oder Spezifizität dieses Biomoleküls verbunden [J. Nucl. Med. 24, 1158 (1983)].

Makromoleküle können generell als Kontrastmittel für die Angiographie geeignet sein. Albumin-GdDTPA (Radiology 1987; 162: 205) z.B. zeigt jedoch 24 Stunden nach intravenöser Injektion bei der Ratte eine Anreicherung im Lebergewebe, die fast 30 % der Dosis ausmacht. Außerdem werden in 24 Stunden nur 20 % der Dosis eliminiert.
Das Makromolekül Polylysin-GdDTPA (Europäische Patentanmeldung, Publikations-Nr. 0 233 619) erwies sich ebenfalls geeignet als blood-pool-agent. Diese Verbindung besteht jedoch herstellungsbedingt aus einem Gemisch von Molekülen verschiedener Größe. Bei Ausscheidungsversuchen bei der Ratte konnte gezeigt werden, daß dieses Makromolekül unverändert durch glomeruläre Filtration über die Niere ausgeschieden wird. Synthese-bedingt kann Polylysin-GdDTPA aber auch Makromoleküle enthalten, die so groß sind, daß sie bei der glomerulären Filtration die Kapillaren der Niere nicht passieren können und somit im Körper zurückbleiben.

Auch makromolekulare Kontrastmittel auf der Basis von Kohlenhydraten, z.B. Dextran, sind beschrieben worden (Europäische Patentanmeldung, Publikations-Nr. 0 326 226).
Der Nachteil dieser Verbindungen liegt darin, daß diese in der Regel nur ca. 5 % des signalverstärkenden paramagnetischen Kations tragen.

Es bestand daher die Aufgabe, neue diagnostische Mittel vor allem zur Erkennung und Lokalisierung von Gefäßkrankheiten, die die genannten Nachteile nicht besitzen, zur Verfügung zu stellen. Diese Aufgabe wird durch die vorliegende Erfindung gelöst.

Die erfindungsgemäßen Polyrotaxane lassen sich durch die allgemeine Formel I beschreiben 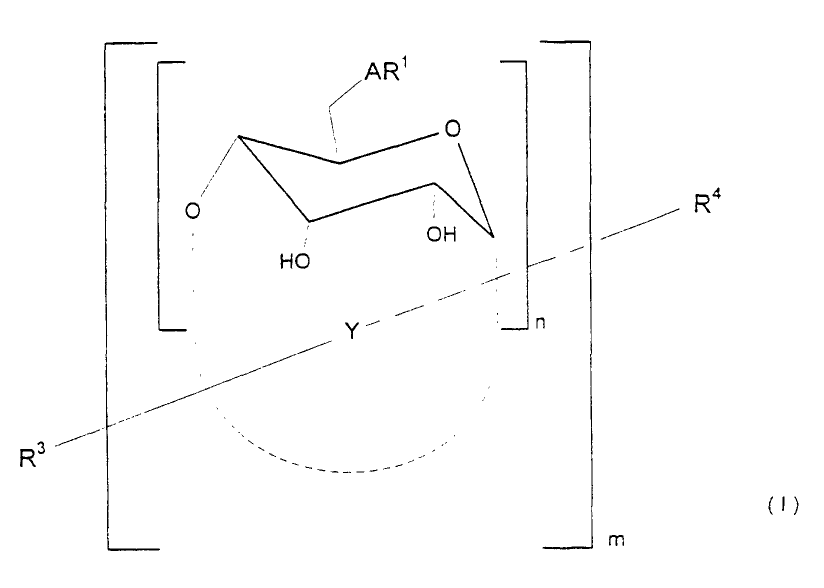 worin
- n: die Zahlen 6,7 oder 8,
- m: die Zahlen 2 bis 50,
- A: ein Sauerstoffatom oder die Gruppe -XNH-,
worin
X eine direkte Bindung oder den Rest -O-(CO)ₓ-CHR-(CH₂)_{y}- mit x in der Bedeutung der Zahlen 0 oder 1 und
y in der Bedeutung der Zahlen 0 bis 10
bedeutet,
- R¹: die kontrastgebenden Reste II, III, IV, V, VI, VII, VIII, IX oder X 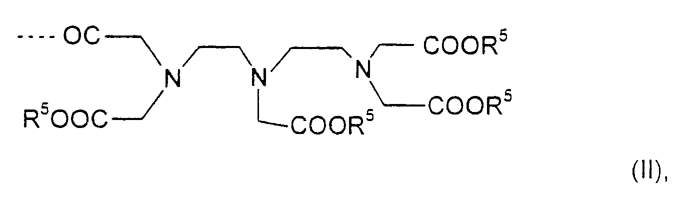 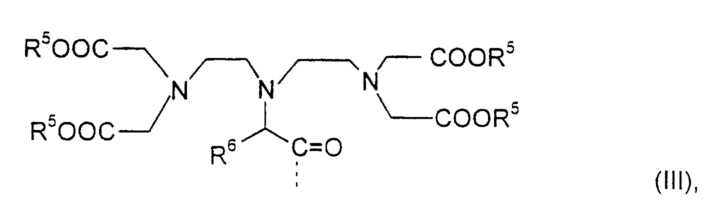 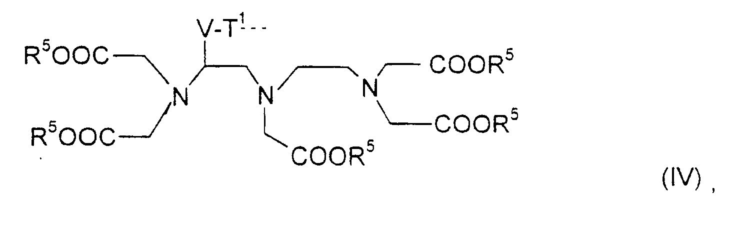 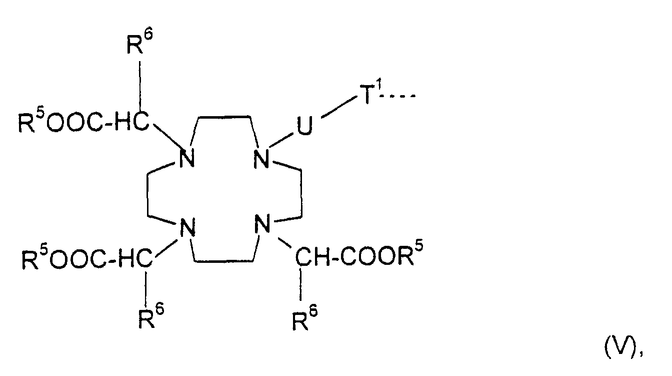 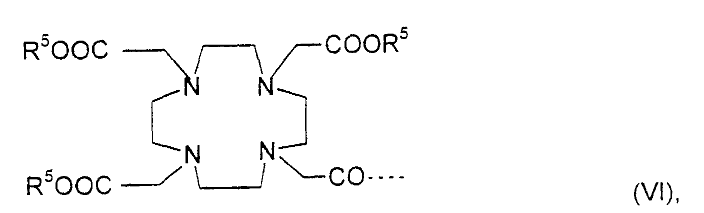 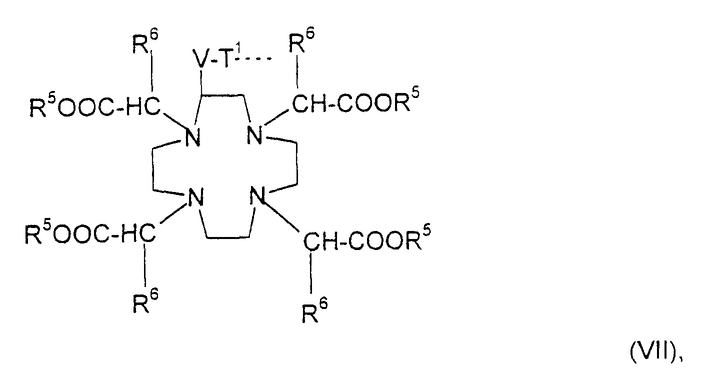 worin
R⁵ unabhängig voneinander ein Wasserstoffatom oder ein Metallionenäquivalent der
Elemente der Ordnungszahlen 20-32, 37 - 39, 42 - 44, 49 oder 57 -83,
R⁶ ein Wasserstoffatom, einen geradkettigen oder verzweigten C₁-C₇-Alkylrest, einen Phenyl- oder Benzylrest,
- V: eine -CH₂-(CH₂)ₒ-(O)ₚ 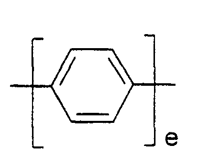 gruppe mit
- o: in der Bedeutung der Zahlen 0 bis 10,
- p: und e jeweils in der Bedeutung der Ziffern 0 oder 1, mit der Maßgabe, daß p nur dann für die Ziffer 1 steht, wenn e die Ziffer 1 bedeutet,
- T¹: eine -NHCS- oder -CO-gruppe,
- U: eine -CHR⁷-CONR^{7'}-M¹- oder -CH₂-CH(OH)-M²-gruppe mit R⁷ und R^{7'} unabhängig voneinander in der Bedeutung von R⁶ oder der Gruppe -CH₂-(CH₂)ₒ-COOH und M¹ und M² jeweils in der Bedeutung eines Phenylenrestes oder einer geradkettigen, verzweigten, gesättigten oder ungesättigten C₁-C₂₀-Alkylenkette, die gegebenenfalls substituiert ist durch 1-5 (CH₂)ₒ-COOH, 1-5 OR⁶-reste oder 1-8 Sauerstoffatome, 1-2-NH-, 1-2-C(=NH)-, 1-5-CONR⁷, 1-5-NR⁷CO-, 1-2 Phenylen- oder 1-2 Phenylenoxygruppen enthält, bedeuten, mit der Maßgabe, daß mindestens zwei der Reste R⁵ für Metallionenäquivalente der Elemente der oben genannten Ordnungszahlen stehen sowie gegebenenfalls Kationen anorganischer und/oder organischer Basen, Aminosäuren oder Aminosäureamide, 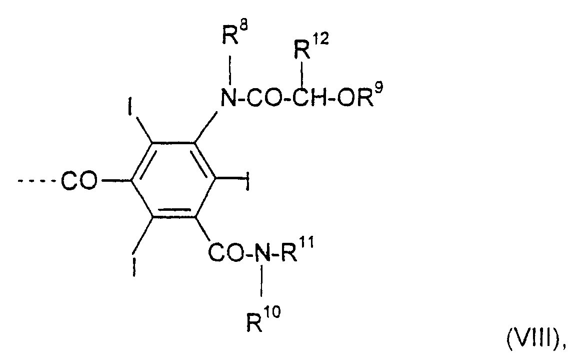 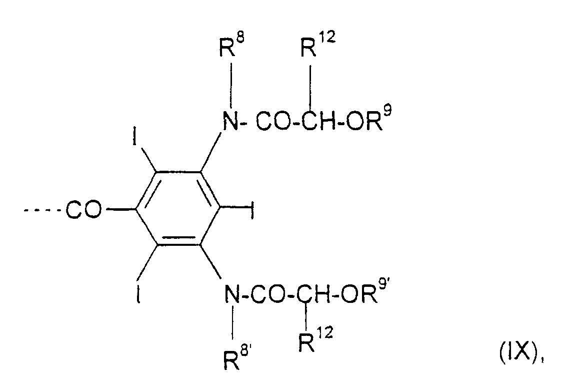 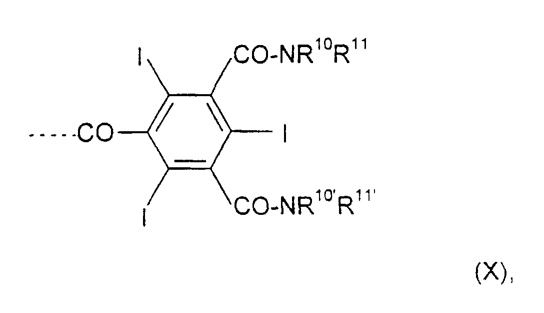 mit
R⁸, R^{8'}, R¹⁰, R^{10'}, R¹¹, R^{11'}, die gleich oder verschieden sein können, in der Bedeutung Wasserstoff oder geradkettiges Alkyl mit 2-6 C-Atomen oder verzweigtkettiges Alkyl mit 3-6 C-Atomen, wobei beide Alkylreste mit 1-5 OH Gruppen substituiert sein können,
R⁹, R^{9'}, R¹², die gleich oder verschieden sein können, in der Bedeutung Wasserstoff oder Methyl und
- Y: die Reste -W -(CH₂)₂[O-CH₂-CH₂]_{q}-W, 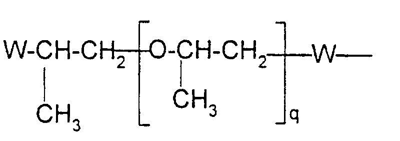 worin q die Zahlen 8 bis 200, W die NH-gruppe oder das O-Atom bedeuten, und
- R³ und R⁴: unabhängig voneinander einen Substituenten mit einem Durchmesser von mindestens 0,6 nm bedeuten.

Als bevorzugte Substituenten für R⁶ und R⁷ bzw. R^{7'} seien das Wasserstoffatom und die Methylgruppe genannt. Als bevorzugte Substituenten von R⁸, R^{8'}, R¹⁰, R^{10'}, R¹¹ und R^{11'} seien Wasserstoff, die Methylgrupppe sowie geradkettige oder verzweigte Alkylgruppen mit bis zu 6 C-Atomen, die durch 1 - 5 Hydroxygruppen substituiert sein können, genannt. Als Beispiele für letztere seien angeführt:

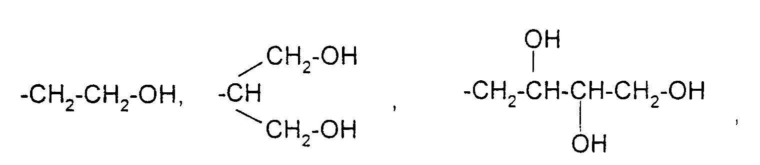

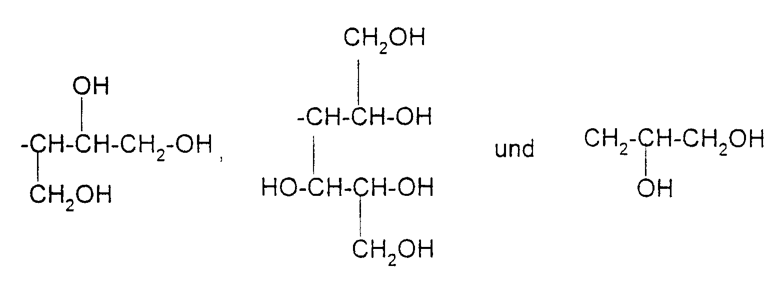

Für q ist der bevorzugte Bereich 15 - 80.

Für w ist der bevorzugte Bereich 5 - 30.

Als für V stehende bevorzugte Gruppen seien beispielhaft genannt die CH₂C₆H₄-, CH₂-O-C₆H₄-, (CH₂)₄-, (CH₂)₆- und (CH₂)₁₀-Gruppe, wobei die C₆H₄-gruppe an T¹ gebunden ist.

Bevorzugte Substituenten für R⁷ sind das Wasserstoffatom, die Methyl-, CH₂COOH- und (CH₂)₂COOH-Gruppe.

Als Gruppen M¹ seien beispielhaft genannt

α-(CH₂)₁₋₁₀-β, α-CH(CH₃)-β,

α-CH[CH₂CH(CH₃)₂]-β, α-CH-(C₆H₅)-β

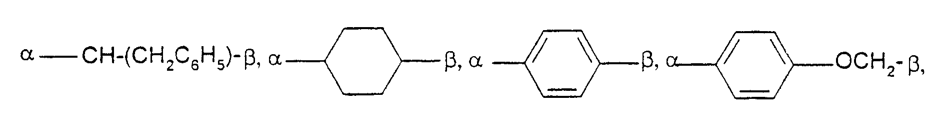

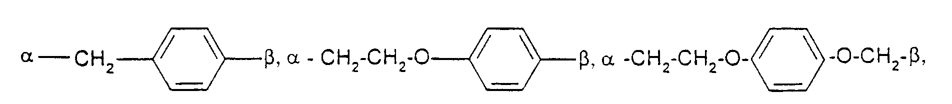

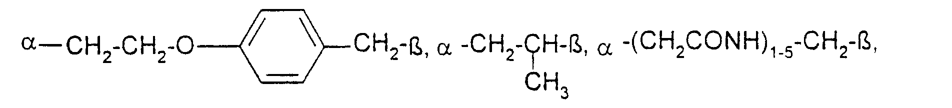

α-CH₂-CH₂-O-CH₂-β, α-(CH₂)₂₋₃-(OCH₂CH₂)₁₋₅-O-(CH₂)₂₋₃-β,

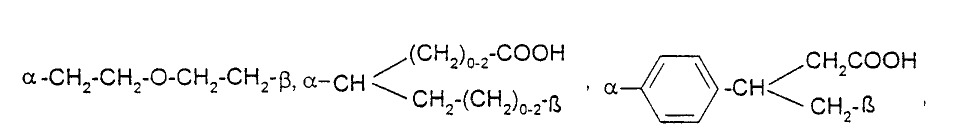

wobei α die Verknüpfungsstelle an den Rest-CONR⁷ und β die Verknüpfungsstelle an T¹ angibt.

Bevorzugt sind die Gruppen:

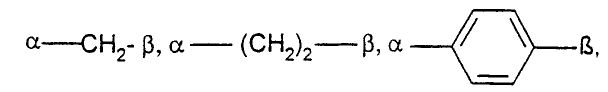

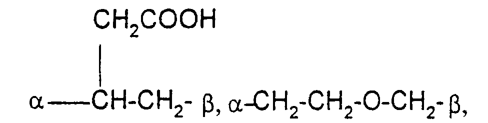

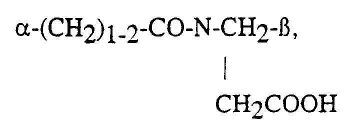

α-CH₂-CH₂-CO-NH(CH₂)₆-β, α-(CH₂)₅-β,

Als Gruppen M² seien beispielhaft genannt: 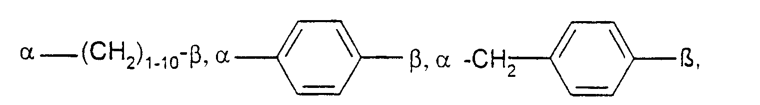 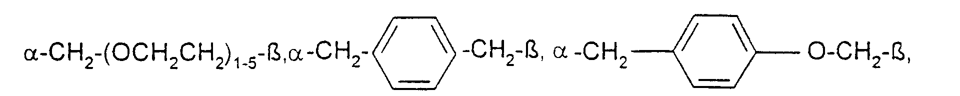 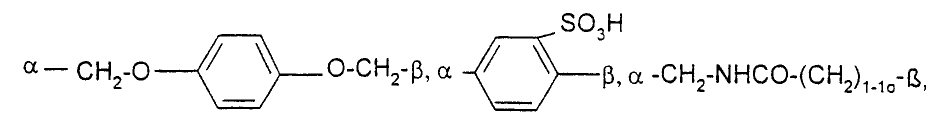 wobei α die Verknüpfungsstelle an den Rest-CH(OH)- und β die Verknüpfungsstelle an T¹ angibt.

Bevorzugt sind die Gruppen:

α -CH₂-β,

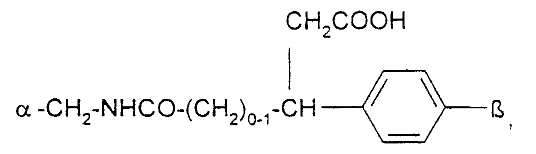

α-CH₂-NHCO-(CH₂)₂₋₃-β

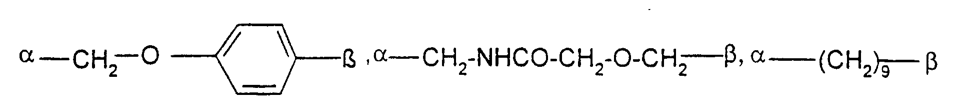

Besonders bevorzugt ist die Gruppe:

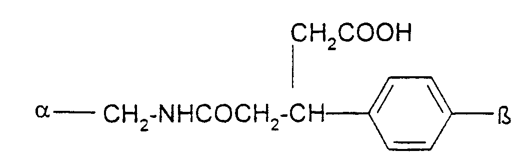

Als bevorzugte Reste R¹ bzw. R³ und R⁴ seien die kontrastgebenden Reste II, IIIa, V - insbesondere Va, Vb, Vc und Vd -, VIIIa, VIIIb 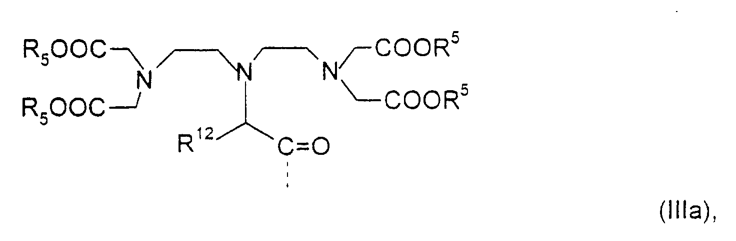 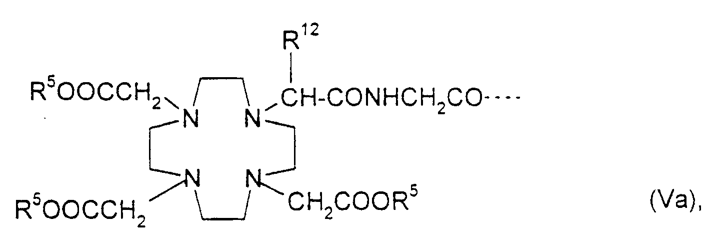 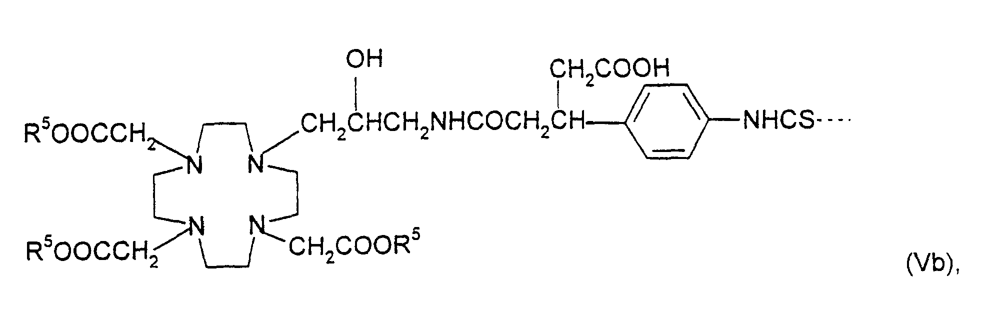 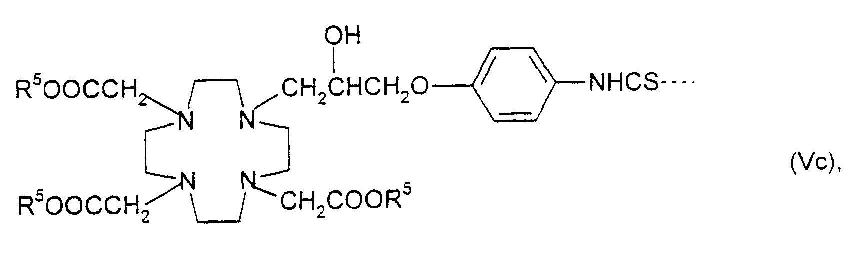 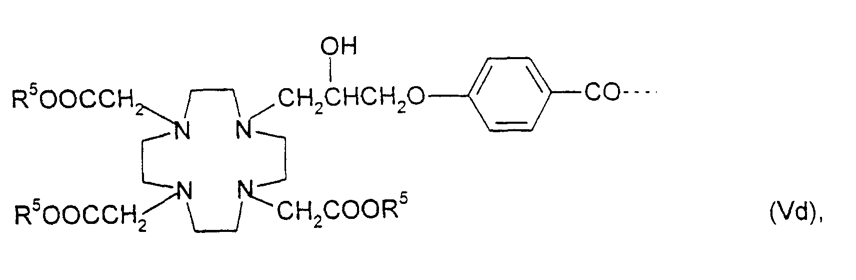 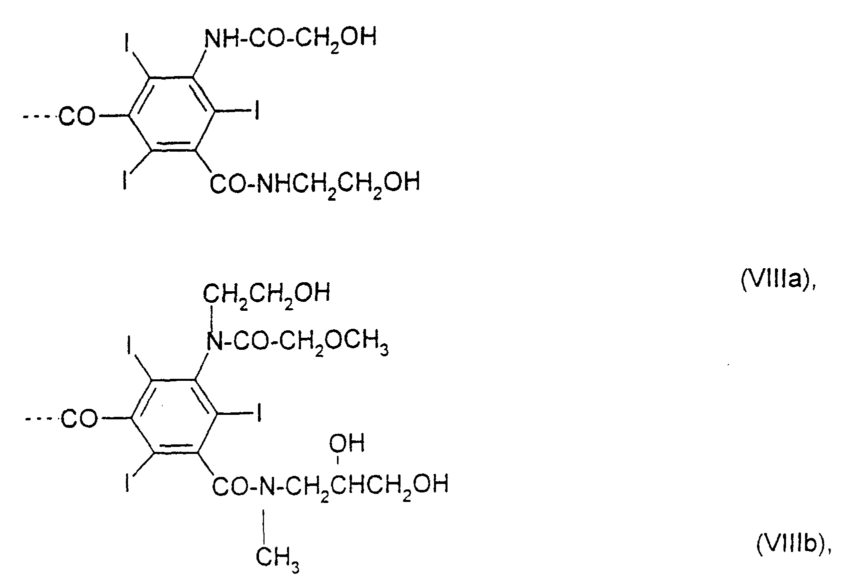 genannt.

Als weitere bevorzugte Reste für R³ und R⁴ seien die Reste der allgemeinen Formel 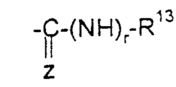 mit Z in der Bedeutung eines Sauerstoffatoms
,oder Schwefelatoms und r in der Bedeutung der Zahlen 0 oder 1, insbesondere

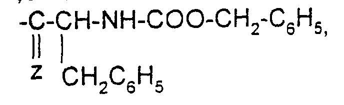

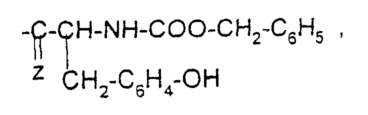

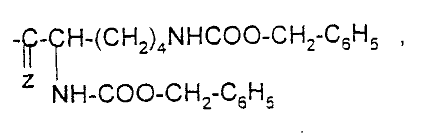

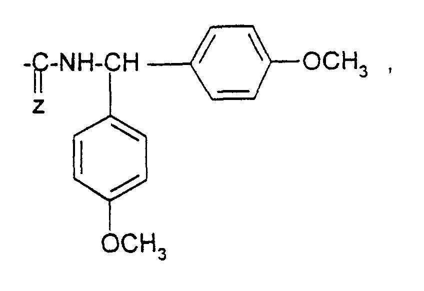

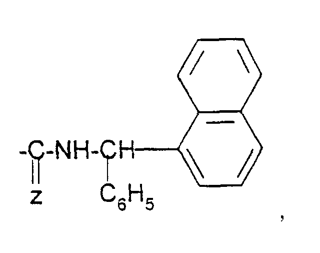

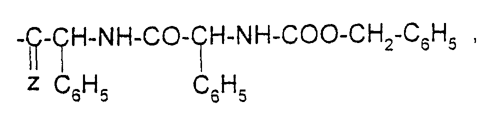

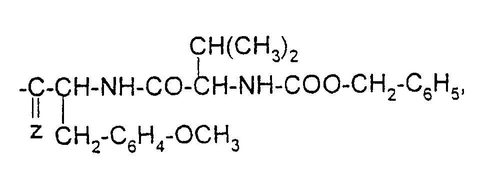

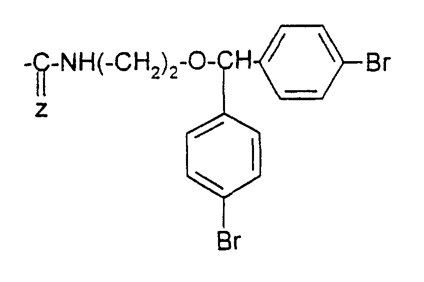

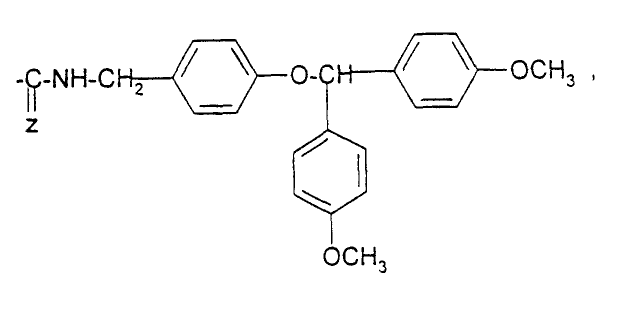

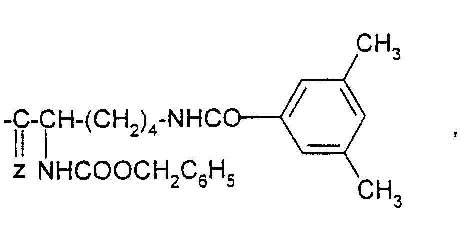

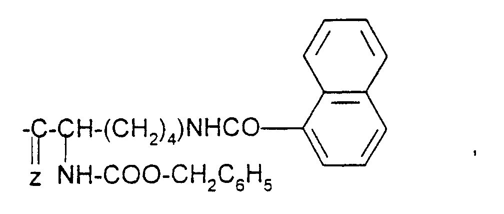

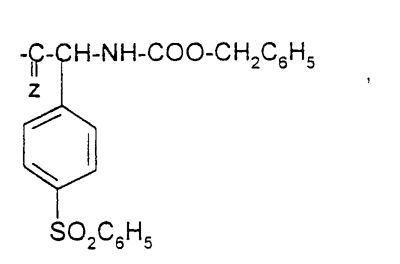

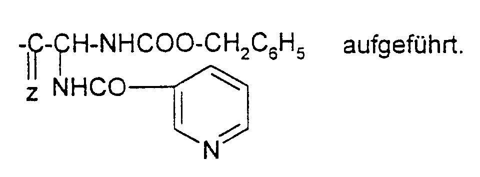

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21 - 29, 42, 44 und 58 - 70. Geeignete Ionen sind beispielsweise das Chrom(III)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)-, Mangan(II)- und Eisen(III)-ion.

Ist die erfindungsgemäße Verbindung zur Anwendung in der Röntgen-Diagnostik bestimmt, so leitet sich das Metallion vorzugsweise von einem Element höherer Ordnungszahl ab, um eine ausreichende Absorption der Röntgenstrahlen zu erzielen. Es wurde gefunden, daß zu diesem Zweck diagnostische Mittel, die ein physiologisch verträgliches Komplexsalz mit Metallionen von Elementen der Ordnungszahlen 25 und 26 sowie 57 - 83 enthalten, geeignet sind.

Bevorzugt sind Mangan(II)-, Eisen(II)-, Eisen(III)-, Praseodym(III)-, Neodym(III)-, Samarium(III)-, Gadolinium(III)-, Ytterbium(III)- oder Bismut(III)-ionen, insbesondere Dysprosium(III)-ionen.

Die erfindungsgemäßen Polyrotaxan-Komplexe enthalten mindestens 12 Ionen eines Elements der oben genannten Ordnungszahl.

Die restlichen aciden Wasserstoffatome, das heißt diejenigen, die nicht durch das Zentralion substituiert worden sind, können gegebenenfalls ganz oder teilweise durch Kationen von anorganischen und/oder organischen Basen, Aminosäuren oder Aminosäure-amiden ersetzt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die erfindungsgemäßen Verbindungen, die ein Molekulargewicht von 10.000 - 200.000, vorzugsweise 20.000 - 80.000 Da, besitzen, weisen die eingangs geschilderten gewünschten Eigenschaften auf. Sie enthalten die für ihre Verwendung benötigte große Anzahl von Metallionen im Komplex stabil gebunden.

Die neuen Polyrotaxane reichern sich in Gebieten mit erhöhter Gefäßpermeabilität, wie z.B. in Tumoren, an, erlauben Aussagen über die Perfusion von Geweben, geben die Möglichkeit, das Blutvolumen in Geweben zu bestimmen, die Relaxationszeiten bzw. Densitäten des Blutes selektiv zu verkürzen, und die Permeabilität der Blutgefäße bildlich darzustellen. Solche physiologischen Informationen sind nicht durch den Einsatz von extrazellulären Kontrastmitteln, wie z.B. Gd-DTPA [Magnevist®], zu erhalten. Aus diesen Gesichtspunkten ergeben sich auch die Einsatzgebiete bei den modernen bildgebenden Verfahren Kernspintomographie und Computertomographie: spezifischere Diagnose von malignen Tumoren, frühe Therapiekontrolle bei zytostatischer, antiphlogistischer oder vaso-dilatativer Therapie, frühe Erkennung von minderperfundierten Gebieten (z.B. im Myokard), Angiographie bei Gefäßerkrankungen, und Erkennung und Diagnose von (sterilen oder infektiösen) Entzündungen. Außerdem sind die erfindungsgemäßen Polyrotaxan-Komplexe hervorragend zur Darstellung der Lymphgefäße (interstitielle und intravenöse Lymphographie) geeignet .

Als weitere Vorteile gegenüber extrazellulären Kontrastmitteln, wie z.B. Gd-DTPA [Magnevist®], muß die höhere Effektivität als Kontrastmittel für die Kernspintomographie (höhere Relaxivität) hervorgehoben werden, was zu einer deutlichen Reduktion der diagnostisch notwendigen Dosis führt. Gleichzeitig können die erfindungsgemäßen Kontrastmittel als Lösungen isoosmolar zum Blut formuliert werden und verringern dadurch die osmotische Belastung des Körpers, was sich in einer verringerten Toxizität der Substanz (höhere toxische Schwelle) niederschlägt. Geringere Dosen und höhere toxische Schwelle führen zu einer signifikanten Erhöhung der Sicherheit von Kontrastmittelanwendungen bei modernen bildgebenden Verfahren.

Im Vergleich zu anderen makromolekularen Kontrastmitteln auf der Basis von Kohlenhydraten, z.B. Dextran (Europäische Patentanmeldung, Publikations-Nr. 0 326 226), die - wie erwähnt - in der Regel nur ca. 5 % des signalverstärkenden paramagnetischen Kations tragen, weisen die erfindungsgemäßen Polyrotaxan-Komplexe einen Gehalt von in der Regel ca. 10-20% des paramagnetischen Kations auf. Somit bewirken die erfindungsgemäßen Makromoleküle pro Molekül eine sehr viel höhere Signalverstärkung, was gleichzeitig dazu führt, daß die zur Kernspintomographie notwendige Dosis erheblich kleiner ist.

Mit den erfindungsgemäßen Polyrotaxan-Komplexen ist es gelungen, hochmolekulare Kontrastmittel zur Verfügung zu stellen, die überraschenderweise vollständig eliminiert werden, obwohl das durchschnittliche Molekulargewicht z.T. deutlich über der Nierenfiltrationsschwelle liegt.

Im Vergleich zu den anderen erwähnten Polymer-Verbindungen des Stands der Technik zeichnen sich die erfindungsgemäßen Komplexe durch verbessertes Ausscheidungsverhalten, höhere Wirksamkeit, größere Stabilität und/oder bessere Verträglichkeit aus.

Ein weiterer Vorteil der vorliegenden Erfindung liegt darin, daß nunmehr Komplexe mit hydrophilen oder lipophilen, makrocyclischen oder offenkettigen, niedermolekularen oder hochmolekularen Liganden zugänglich geworden sind. Dadurch ist die Möglichkeit gegeben, Verträglichkeit und Pharmakokinetik dieser Polymer-Komplexe durch chemische Substitution zu steuern.

Die Herstellung der erfindungsgemäßen Polyrotaxan-Komplexe erfolgt dadurch, daß man Verbindungen der allgemeinen Formel II, 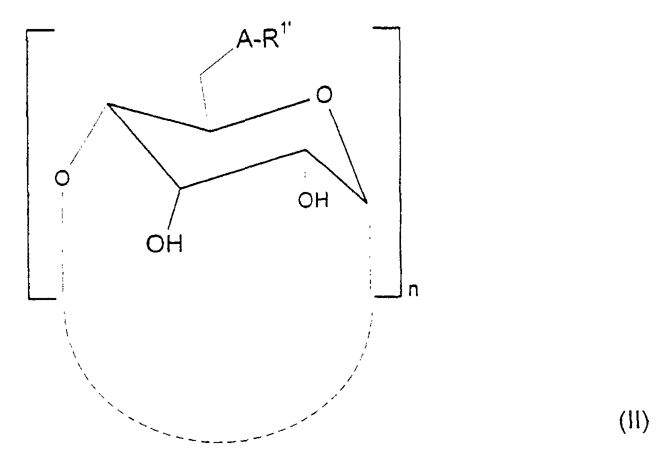 worin
A und n die oben angegebenen Bedeutungen haben und
R^{1'} für ein Wasserstoffatom oder den Rest II', III', IV', V', VI', VII', VIII', IX' oder X', worin diese die für II - X angegebene Bedeutung haben, jedoch die in ihnen vorhandenen Reste R⁵ für Wasserstoff oder Säureschutzgruppen stehen und gegebenenfalls vorhandene Hydroxygruppen gegebenenfalls in geschützter Form vorliegen, steht
mit Verbindungen der allgemeinen Formel XI

H-Y-H (XI)

zu Verbindungen der allgemeinen Formel XII umsetzt 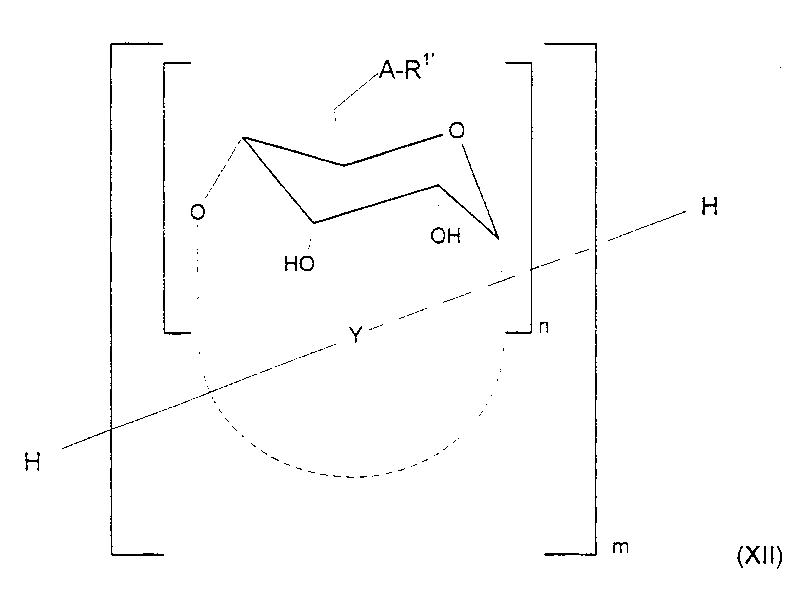 und diese mit einer Verbindung R^{3'}-Fg und/oder R^{4'}-Fg umsetzt,
worin R^{3'} und R^{4'} die für R³ und R⁴ angegebene Bedeutung haben, jedoch die in R¹ stehenden Reste R⁵ für Wasserstoff, Säureschutzgruppen oder ein Metallionenäquivalent der Elemente der genannten Ordnungszahlen und Fg für Anhydrid, ―Cl, ―F, ―OH, ―N₃, 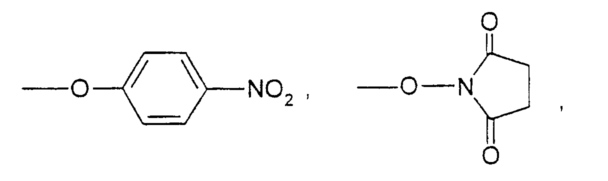 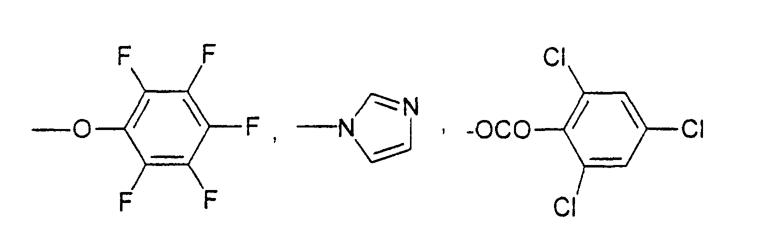 steht,
wobei im Falle, daß R³ und/oder R⁴ in (I) für einen Rest 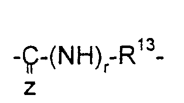 steht, eine Verbindung der allgemeinen Formel R³-N=C=Z und/oder R⁴-N=C=Z eingesetzt wird sowie im Falle, daß in IV, V und VII T¹ für eine NHCS-Gruppe steht, eine Verbindung der allgemeinen Formel IV'', V" oder VII' 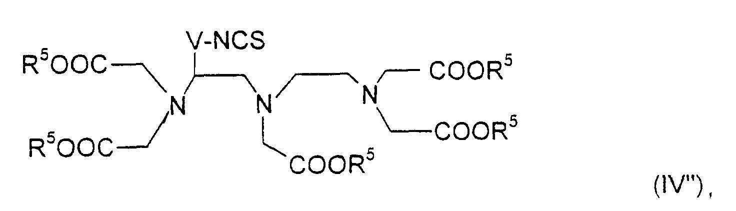 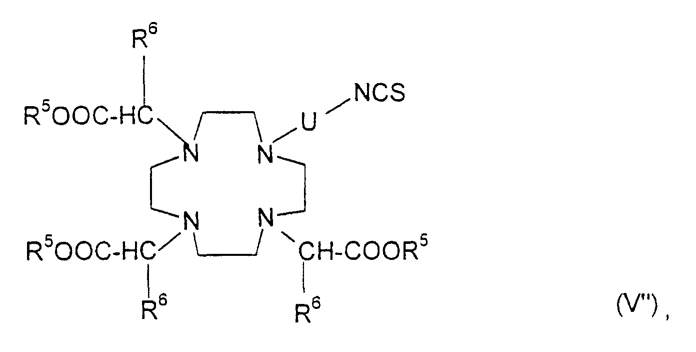 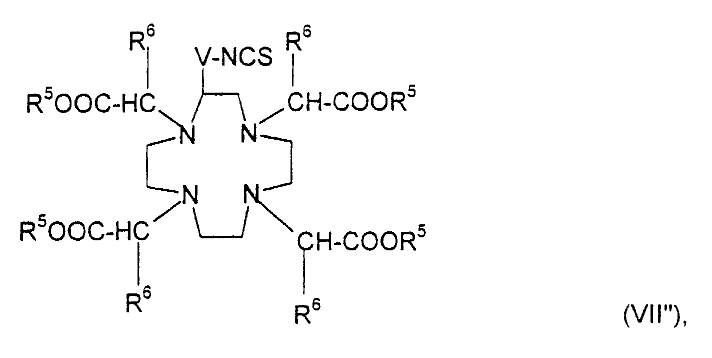 eingesetzt wird, die so erhaltene Verbindung der allgemeinen Formel XIII 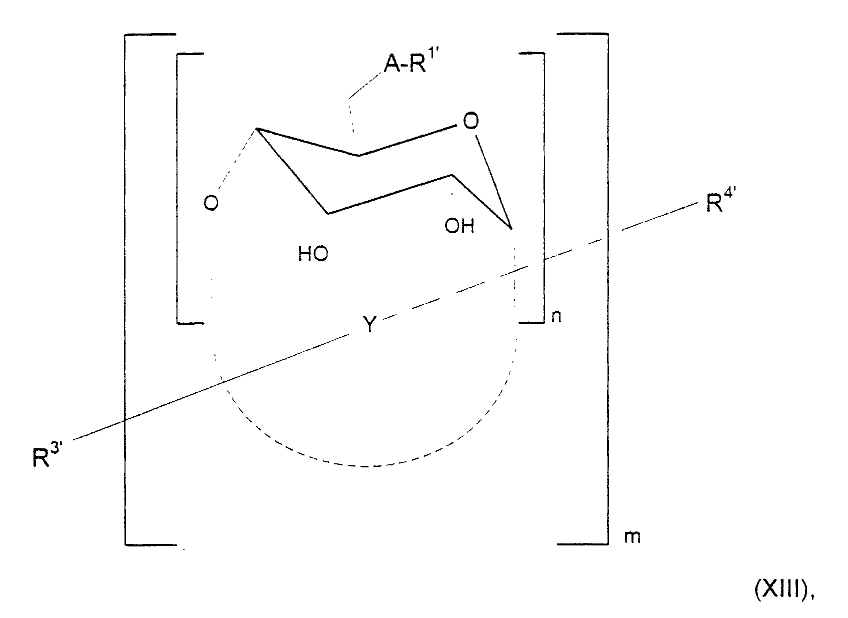 falls R^{1'} die Bedeutung von Wasserstoff hat, mit einer Verbindung R^{1''}-Fg, worin R^{1''} die für R¹ angegebene Bedeutung hat, jedoch die in ihnen vorhandenen Reste R⁵ für Wasserstoff, Säureschutzgruppen oder ein Metallionenäquivalent der Elemente der genannten Ordnungszahlen stehen, bzw. mit Verbindungen der allgemeinen Formel IV'', V'' oder VII'' umsetzt und anschließend, für den Fall, daß R⁵ in XIII nicht für die o.g. Metallionenäquivalente steht, gegebenenfalls vorhandene Säureschutzgruppen abspaltet, die gewünschten Metallionen einführt und anschließend, falls gewünscht, vorhandene acide Wasserstoffatome durch Kationen anorganischer und/oder organischer Basen, Aminosäuren oder Aminosäureamide substituiert.

Die Herstellung der Pseudopolyrotaxane der allgemeinen Formel XII erfolgt nach literaturbekannten Methoden (z.B. A. Harada et al., J.Am. Chem. Soc. 1994, 116, 3192 - 96; A. Harada et al., Nature, 370, 126 - 128 (1994); Y.-M. Jeon et al., Chemistry Letters 1996, 503 - 504), indem man das fadenförmige Molekül der allgemeinen Formel XI in einem polaren Lösungsmittel, wie z.B. Wasser, DMSO, Fornamid sowie deren Mischungen, vorzugsweise Wasser, bei Temperaturen zwischen 10 und 100 °C, vorzugsweise bei Raumtemperatur, gegebenenfalls im Ultraschall-Bad, gegebenenfalls unter Zusatz von anorganischen Salzen, wie z.B. Natriumchlorid, Kaliumchlorid, Natriumbromid, mit dem gewünschten Cyclodextrin der allgemeinen Formel II umsetzt.

Die nachfolgende Umwandlung der Pseudopolyrotaxane in die gewünschten Polyrotaxane der allgemeinen Formel XIII erfolgt, wie auch in den Beispielen beschrieben, nach literaturbekannten Methoden (H.W. Gibson et al., Adv. Mater. 1993, 5, 11 - 21; H.W. Gibson et al., J. Org. Chem. 1993, 58, 3748 - 56; H.W. Gibson et al., J. Am. Chem. Soc. 1995, 117, 852 - 874; D.B. Amabilino et al., Am. Chem. Soc. 1996, 118, 12.012 - 20; I. Yamaguchi et al., Am. Chem. Soc. 1996, 118, 1811 - 12), durch Umsetzung mit Verbindungen der allgemeinen Formel R^{3'}-Fg und/oder R^{4'}-Fg bzw. mit Verbindungen der allgemeinen Formel R³-N=C=Z bzw. und/oder R⁴-N=C=Z bzw. mit Verbindungen der allgemeinen Formel IV'', V" oder VII'', vorzugsweise in polaren Lösungsmitteln wie z.B. DMSO, DMF, Wasser oder deren Mischungen bei Temperaturen zwischen 0 und 100 °C, vorzugsweise 10 bis 30 °C, gegebenenfalls unter Zusatz von dem Fachmann bekannten Hilfsstoffen zur Esterbildung (Houben Weyl, Methoden der organischen Chemie, 1985, Bd. E5, S. 656- 772; Comprehensive organic Transformations, Richard Larock. 1989 VCH Publishers Inc. S. 966 - 972), Amidkupplung (Houben Weyl. Methoden der organischen Chemie, 1974, Bd. 15/2, S. 1 - 395; Houben Weyl. Methoden der organischen Chemie, 1985, Bd. E5, S. 934- 1116; Comprehensive organic Transformations, Richard Larock, 1989 VCH Publishers Inc. S. 972 - 976, Fournic-Zaluski et al. J. Med. Chem. 1996, 39, 2596; Y.M. Angell et al., Tetrahedron Letters 1994, 35, 5981; L.A. Carpino et al., J. Chem. Soc. Commun. 1994, 201; H-O. Kim et al, Tetrahedron Letters 1995, 36, 6013; D. Papaioannou et al, Tetrahedron Letters, 1995, 36, 5187, G. Stemple et all, Bioorg. Med. Letters 1996, 6, 55), Thiocyanatbildung (The chemistry of cyanates and their thio derivatives, Part 2, S. 1003 - 1223, Saul Patai, 1977, John Wiley and Sons; Chem. Soc. Rev. 4, 231 - 250 (1975) und Carbamatbildung (The chemistry of cyanates and their thio derivatives, Part 2, S. 620 - 792, Saul Patai, 1977, John Wiley and Sons). Für den Fall, daß die so synthetisierten Polyrotaxane der allgemeinen Formel XIII im Cyclodextrinteil noch keine Komplexe bzw. Komplexbildner enthalten (d.h. im Falle von R^{1'} = H) werden diese unter den oben für die Herstellung der Polyrotaxane aus den Pseudopolyrotaxanen angegebenen Bedingungen mit Verbindungen der allgemeinen Formel R^{1''}-Fg bzw. mit Verbindungen der allgemeinen Formel IV", V'' oder VII'' umgesetzt.

Für den Fall, daß die in R¹ enthaltenen Reste noch kein Metallion der Elemente der genannten Ordnungszahlen enthalten, werden diese, nach Abspaltung gegebenenfalls vorhandener Säureschutzgruppen, nach dem Fachmann bekannten Methoden (z.B. EP 071564) eingeführt.

Stehen die Reste R³ und/oder R⁴ für einen Rest in der Bedeutung von R¹, können, falls im Edukt der allgemeinen Formel XII der Substituent R^{1'} für Wasserstoff steht, die kontrastgebenden Reste II - X gewünschtenfalls in einer Eintopfreaktion gleichzeitig in das Molekül eingeführt werden.

Die Edukte der allgemeinen Formel II mit R^{1'} in der Bedeutung von Wasserstoff sind Kaufware bzw. literaturbekannt, s. z.B. J. Boger et al, Helv. Chim. Acta 61, 2190 (1978); Peter R. Ashton et al., J. Org. Chem. 1996, 61, 903 - 908.

Die Verbindungen der allgemeinen Formel II mit R^{1'} in der Bedeutung der Reste II' - X' sind literaturbekannt oder analog literaturbekannten Methoden erhältlich:
- IV":: s.z.B. WO 91/14459,
- V":: s. z.B. US-5,053,503, WO 96/02669, WO 96/01655, EP 0430863, EP 255471, US-5,277,895, EP 0232751, US-4,885, 363,
- VII":: s.z.B. US-4,923,985,

Die Verbindungen der allgemeinen Formel R^{1''}-Fg, d.h. Verbindungen der allgemeinen Formel IIA - XA 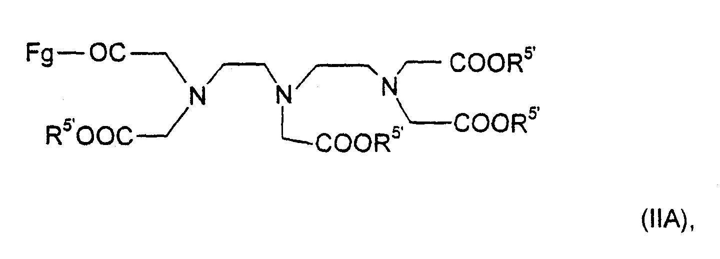 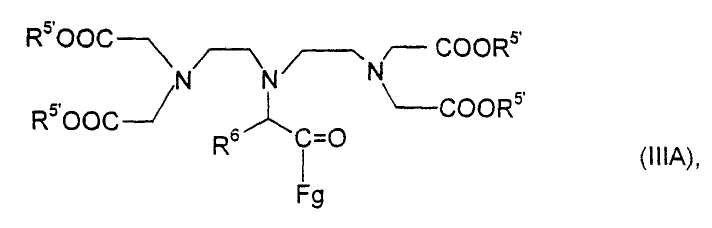 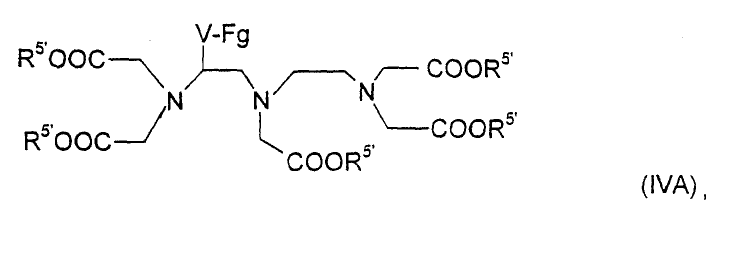 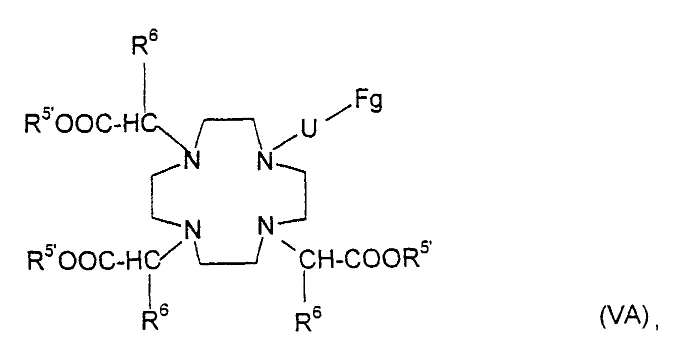 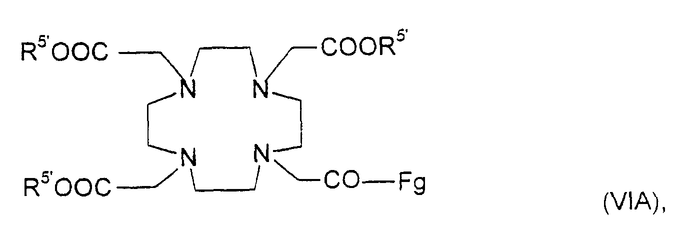 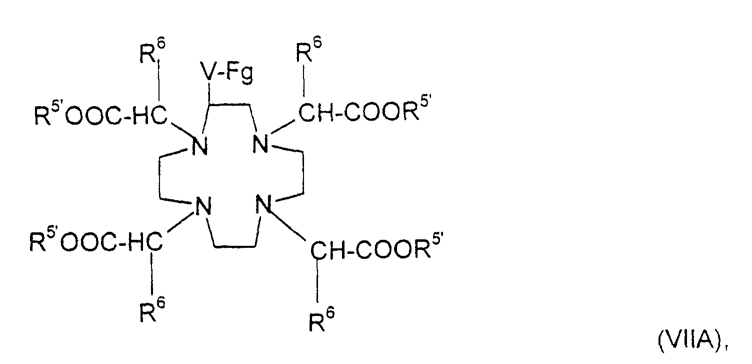 worin
R^{5'} für R⁵ oder eine Säureschutzgruppe steht, 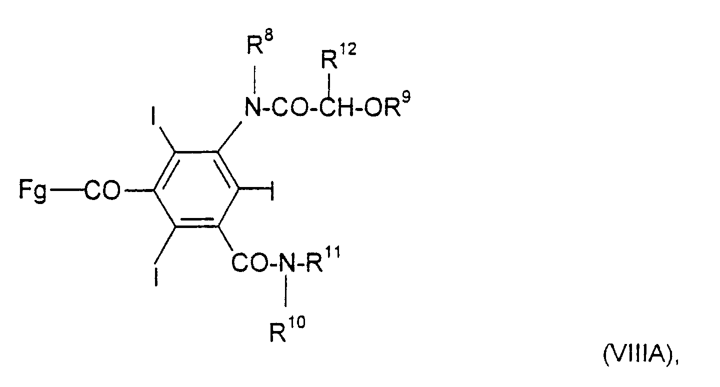 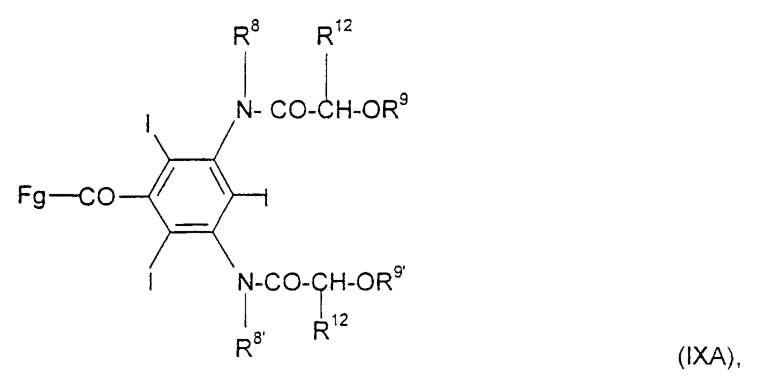 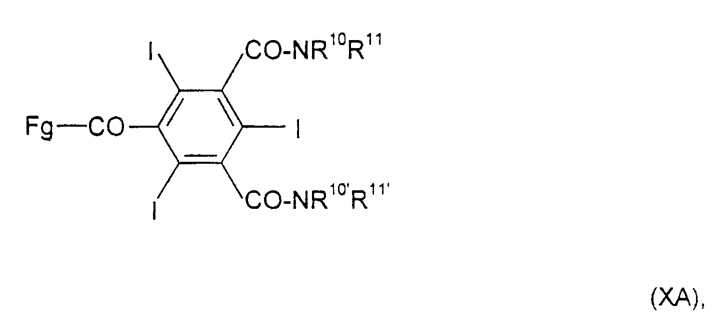 sind literaturbekannt oder analog literaturbekannten Methoden erhältlich:
- IIA:: s. z.B. EP 263059
- IIIA:: s.z.B. DE 19507822, DE 19580858, DE 19507819
- IVA:: s.z.B. WO 91/14459
- VA:: s.z.B. US-5,053,503, WO 96/02669, WO 96/01655, EP 0430863, EP 255471, US-5,277,895, EP 0232751, US-4,885,363
- VIA:: s.z.B. EP 0255471
- VIIA:: s.z.B. US-4,923,985
- VIIIA:: s.z.B. DE 2207950
- IXA:: s.z.B. US-3,702,866
- XA:: s.z.B. EP 0032388

Die fadenförmigen Verbindungen der Formel XI sind kommerziell erhältlich, beispielsweise bei den Firmen Shearwater Polymers Inc., USA. Sigma, Aldrich, Fluka.

Die Reinigung der erhaltenen Polyrotaxan-Komplexe erfolgt gegebenenfalls nach Einstellung der pH-Wertes durch Zusatz einer Säure oder Base auf pH 6 bis 8, bevorzugt ca. 7, vorzugsweise durch Ultrafiltration mit Membranen geeigneter Porengröße (z.B. Amicon® XM30, Amicon®YM10, Amicon®YM3) oder Gelfiltration an z.B. geeigneten Sephadex®-Gelen.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die korrespondierende Calcium-Polyrotaxan-Komplexe) oder - falls erforderlich - Elektrolyte wie zum Beispiel Natriumchlorid oder - falls erforderlich - Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methylcellulose, Lactose, Mannit] und/oder Tensid(en) [zum Beispiel Lecithine, Tween®, Myrj®] und/oder Aromastoff(en) zur Geschmackskorrektur [zum Beispiel ätherischen Ölen] gemischt.

Die erfindungsgemäßen pharmazeutischen Mittel enthalten vorzugsweise 1µMol - 1 Mol/l des Komplexsalzes und werden in der Regel in Mengen von 0,0001 - 5 mMol/kg dosiert. Sie sind zur enteralen und parenteralen Applikation bestimmt. Die erfindungsgemäßen Komplexverbindungen kommen zur Anwendung für die NMR- und Röntgen-Diagnostik in Form ihrer Komplexe mit den Ionen der Elemente mit den Ordnungszahlen 21-29, 39, 42, 44 und 57-83.

Die erfindungsgemäßen Mittel erfüllen die vielfältigen Voraussetzungen für die Eignung als Kontrastmittel für die Kernspintomographie. So sind sie hervorragend dazu geeignet, nach enteraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100- bis 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,001 - 5 mMol/kg, vorzugsweise 0,005 - 0,5 mMol/kg, dosiert. Details der Anwendung werden zum Beispiel in H.-J. Weinmann et al., Am. J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen therapeutischen Mittel werden parenteral, vorzugsweise i.v., appliziert.

Im allgemeinen werden die erfindungsgemäßen Mittel für die Anwendung als Röntgenkontrastmittel in Analogie zu z.B. Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler "Einführung in die Röntgendiagnostik", G. Thieme, Stuttgart. New York (1977) diskutiert.

Insgesamt ist es gelungen, neue Metallkomplex- und jodhaltige Polyrotaxane zu synthetisieren, die neue Möglichkeiten in der Diagnostik erschließen.

Die nachfolgenden Beispiele dienen zur näheren Erläuterung des Erfindungsgegenstands:

Im Text werden folgende Abkürzungen verwendet:

| | |
|---|---|
| Gadolinium-GlyMeDOTA-Säure | Gadolinium-Komplex der 10-[4-Carboxy-1-methyl-2-oxo-3-azabutyl]-1,4,7,10-tetraazacyclododecan-1,4,7-triessigsäure |
| DTPA(t-butyl)₄-monocarbonsäure | 3,9-Bis-(tert.-butoxycarbonylmethyl)-6-carboxymethyl-3,6,9-triazaundecan-di-tert.-butylester |

### Beispiel 1

### a) N-(2-Brompropionyl)-glycinbenzylester

Zu 100 g (296,4 mmol) Glycinbenzylester p-Toluolsulfonsäuresalz und 89,98 g (889,2 mmol) Triethylamin in 500 ml Methylenchlorid tropft man bei 0°C 60,97 g (355,7 mmol) α-Brompropionylchlorid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 500 ml 5 %iger aqu. Salzsäure extrahiert, über Magnesiumsulfat getrocknet und im Vakuum zur Trockne eingedampft. Der Rückstand wird aus Di-isopropylether umkristallisiert. Ausbeute: 69,39 g (78 % d. Th.)

| Elementaranalyse: | | | | |
|---|---|---|---|---|
| ber. | C 48,02 | H 4,70 | N 4,67 | Br 26,62 |
| gef. | C 47,91 | H 4,82 | N 4,51 | Br 26,47 |

### b) 1-[4-Benzyloxycarbonyl)-1-methyl-2-oxo-3-azabutyl]-1,4,7,10-tetraazacyclododecan

Zu 86,14 g (500 mmol) 1,4,7,10-Tetraazacyclododecan gelöst in 1000 ml Chloroform gibt man 50 g (166,6 mmol) der Titelverbindung aus Beispiel 1a und rührt 24 Stunden bei Raumtemperatur. Man extrahiert 3 mal mit 600 ml Wasser, trocknet die organische Phase über Magnesiumsulfat und dampft im Vakuum zur Trockne ein.
Ausbeute: 53,48 g (82 % d. Th.) eines leicht gelb gefärbten Öls
Wassergehalt: 1,5 %

| Elementaranalyse (berechnet auf wasserfreie Substanz): | | | |
|---|---|---|---|
| ber. | C 61,36 | H 8,50 | N 17,89 |
| gef. | C 62,03 | H 8,75 | N 17,36 |

### c) 10-[4-Benzyloxycarbonyl)-1-methyl-2-oxo-3-azabutyl]-1,4,7,10-tetraazacyclododecan-1,4,7-triessigsäure-tri-tert-butylester, Natriumbromid

Zu 50 g (127,7 mmol) der Titelverbindung aus Beispiel 1b und 54,14 g (510,8 mmol) Natriumcarbonat in 500 ml Acetonitril gibt man 82,20 g (421,4 mmol) Bromessigsäure-tert.butylester und rührt 12 Stunden bei 60°C. Man kühlt auf 0°C und filtriert von den Salzen ab. Das Filtrat wird zur Trockne eingedampft und der Rückstand an Kieselgel chromatographiert (Laufmittel: Methylenchlorid/Methanol = 20:1).
Ausbeute: 90,83 g (85 % d. Th.) eines farblosen Feststoffes

| Elementaranalyse: | | | | | |
|---|---|---|---|---|---|
| ber. | C 54,54 | H 7,59 | N 8,37 | Na 2,75 | Br 9,55 |
| gef. | C 54,37 | H 7,71 | N 8,21 | Na 2,83 | Br 9,69 |

### d) 10-(4-Carboxy-1-methyl-2-oxo-3-azabutyl)-1,4,7,10-tetraazacyclododecan-1,4,7-triessigsäure-tri-tert-butylester (Natriumbromid-Komplex)

90 g (107,5 mmol) der Titelverbindung aus Beispiel 1c löst man in 1000 ml Isopropanol und gibt 5 g Palladiumkatalysator (10 % Pd/C) zu. Man hydriert über Nacht bei Raum-temperatur. Es wird vom Katalysator abfiltriert und das Filtrat zur Trockne eingedampft. Der Rückstand wird aus Dioxan umkristallisiert.
Ausbeute: 77,06 g (96 % d. Th.) eines kristallinen Feststoffes

| Elementaranalyse: | | | | | |
|---|---|---|---|---|---|
| ber. | C 49,86 | H 7,69 | N 9,38 | Na 3,08 | Br 10,70 |
| gef. | C 49,73 | H 7,79 | N 9,21 | Na 3,19 | Br 10,83 |

### e) 10-(4-Carboxy-1-methyl-2-oxo-3-azabutyl)-1,4,7,10-tetraazacyclododecan-1,4,7-triessigsäure

77 g (103,1 mmol) der Titelverbindung aus Beispiel 1d werden in 500 ml Trifluoressigsäure gelöst und 3 Stunden bei Raumtemperatur gerührt. Man dampft zur Trockne ein, nimmt den Rückstand in 300 ml Wasser auf und gibt die Lösung auf eine Säule, gefüllt mit Reillex® 425 PVP. Man eluiert mit Wasser. Die produkthaltigen Fraktionen werden vereinigt und zur Trockne eingedampft, der Rückstand wird aus Methanol/Aceton umkristallisiert.
Ausbeute: 44,04 g (84 % d. Th.) eines farblosen, hygroskopischen Feststoffes
Wassergehalt: 6,5 %

| Elementaranalyse (berechnet auf wasserfreie Substanz): | | | |
|---|---|---|---|
| ber. | C 47,99 | H 6,99 | N 14,73 |
| gef. | C 47,83 | H 7,12 | N 14,55 |

### f) Gadolinium-Komplex der 10-(4-Carboxy-1-methyl-2-oxo-3-azabutyl)-1,4,7,10-tetraazacyclododecan-1,4,7-triessigsäure, Gadolinium-GlyMeDOTA-Säure

Zu 40 g (84,12 mmol) der Titelverbindung aus Beispiel 1e gelöst in 400 ml Wasser, gibt man 15,27 g (42,06 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Dimethylether gewaschen und im Vakuumofen bei 130°C getrocknet (24 Stunden).
Ausbeute: 50,53 g (93 % d. Th.) eines farblosen kristallinen Pulvers
Wassergehalt: 2.5 %

| Elementaranalyse (berechnet auf wasserfreie Substanz): | | | | |
|---|---|---|---|---|
| ber. | C 36,24 | H 4,80 | N 11,12 | Gd 24,97 |
| gef. | C 36,35 | H 4,95 | N 10,98 | Gd 24,80 |

### g) Polyrotaxan aus α-Cyclodextrin und dem PEG-bisamid-derivat aus O,O'-Bis (aminoethyl)-PEG und Gadolinium-GlyMeDOTA-Säure

13 g (13,4 mmol) α-Cyclodextrin werden in 80 ml Wasser gelöst. Nach Zugabe von 1,01 g (0,3 mmol) O,O'-Bis(aminoethyl)-PEG (Sigma) wird 30 Minuten bei Raumtemperatur gerührt, die Suspension anschließend noch 3 Minuten im Ultraschallbad behandelt und über Nacht gerührt. Der Niederschlag wird abgesaugt, mit wenig Wasser gewaschen und bei 50°C im Vakuum getrocknet. Man erhält 4,06 g farbloses Pulver. Gleichzeitig werden 416 mg (0,66 mmol) der in Beispiel 1f beschriebenen Gadolinium-GlyMeDOTA-Säure und 77 mg N-Hydroxysuccinimid in 5 ml Dimethylsulfoxid unter Erwärmen gelöst. Nach Abkühlen auf Raumtemperatur werden 136 mg N,N'-Dicyclohexylcarbodiimid zugesetzt und 60 Minuten gerührt. Zu der so hergestellten N-Hydroxysuccinimidester-Lösung gibt man 4,06 g des oben beschriebenen getrockneten α-Cyclodextrin-pseudopolyrotaxans, gelöst in 50 ml DMSO, und 67 mg (0,66 mmol) Triethylamin und rührt über Nacht. Die Lösung wird anschließend mit Diethylether gefällt. Der Niederschlag wird abfiltriert, mit Diethylether gewaschen und an einer RP-18-Säule chromatographiert (Laufmittel: Gradient aus Acetonitril/Tetrahydrofuran/Wasser).
Ausbeute: 4,2 g eines farblosen amorphen Pulvers
Aus der Elementaranalyse ergibt sich eine Besetzung von 11 α-Cyclodextrinen/PEG.

| Elementaranalyse (berechnet auf wasserfreie Substanz): | | | | |
|---|---|---|---|---|
| ber. | C 46,04 | H 6,75 | Gd 2,06 | N 1,10 |
| gef. | C 46,36 | H 7,13 | Gd 1,90 | N 1,02 |

### h) Polyrotaxan aus dem 6,6',6'',6''',6'''',6'''''-Hexa-Gadolinium-GlyMeDOTA-ester des α-Cyclodextrins und dem PEG-bisamid-derivat aus O.O'-Bis(aminoethyl)-PEG und Gadolinium-GlyMeDOTA-Säure

2,0 g (0,13 mmol) der Titelverbindung aus Beispiel 1g, 8.24 g (13 mmol) der in Beispiel 1f beschriebenen Gadolinium-GlyMeDOTA-säure und 1,34 g (13 mmol) Natriumbromid werden in 150 ml Formamid gelöst und auf 10°C gekühlt. Man gibt 3,30 g (16 mmol) N,N'-Dicyclohexylcarbodiimid und 0,122 g (1 mmol) 4-Dimethylaminopyridin hinzu und rührt 2 Stunden bei 10°C und über Nacht bei Raumtemperatur. Die Lösung wird in 1 1 Aceton gegossen und 3 Stunden gerührt. Der Niederschlag wird abfiltriert, in Wasser gelöst und zur Abtrennung von niedermolekularen Bestandteilen über eine AMICON®-Ultrafiltrationsmembran YM3 (cut off 3000 Dalton) filtriert und anschließend gefriergetrocknet. Man erhält 5,30 g (66 % d. Th.) farbloses flockiges Lyophilisat.
H₂O-Gehalt (Karl-Fischer): 8,0 %
Gd-Bestimmung (AAS): 17,2 %

| Elementaranalyse (berechnet auf wasserfreie Substanz): | | | | |
|---|---|---|---|---|
| ber. | C 39,70 | H 5,20 | N 8,61 | Gd 19,21 |
| gef. | C 39,31 | H 5,44 | N 8,76 | Gd 18,39 |

### Beispiel 2

### a) Polyrotaxan aus α-Cyclodextrin und dem PEG-bisamid-derivat aus O,O'-Bis(glycyl)-PEG und Nα-Benzyloxycarbonyl-phenylalanin

13 g (13,4 mmol) α-Cyclodextrin werden in 80 ml Wasser gelöst. Nach Zugabe von 1,04 g (0,3 mmol) O,O'-Bis(glycyl)-PEG (Shearwater Polymers, Inc.) wird 30 Minuten bei Raumtemperatur gerührt, die Suspension anschließend noch 3 Minuten im Ultraschallbad behandelt und über Nacht gerührt. Der Niederschlag wird abgesaugt, mit wenig Wasser gewaschen und bei 50°C im Vakuum getrocknet. Man erhält 4,77 g farbloses Pulver. Anschließend werden 261 mg (0,66 mmol) Nα-Benzyloxycarbonyl-phenylalanin-N-hydroxysuccinimidester (Bachem), 134 mg (1,32 mmol) Triethylamin und 4,77 g des getrockneten α-Cyclodextrin-pseudopolyrotaxans in 50 ml DMSO gelöst und über Nacht gerührt. Die Lösung wird anschließend mit 500 ml Diethylether versetzt. Der Niederschlag wird abfiltriert, mit Diethylether gewaschen und an einer RP-18-Säule chromatographiert (Laufmittel: Gradient aus Acetonitril/Tetrahydrofuran/Wasser).
Ausbeute: 5,25 g eines farblosen Pulvers
Aus der Elementaranalyse ergibt sich eine Besetzung von 14 α-Cyclodextrinen/PEG.

| Elementaranalyse (berechnet auf wasserfreie Substanz): | | | |
|---|---|---|---|
| ber. | C 47,20 | H 6,73 | N 0,32 |
| gef. | C 46,88 | H 6,91 | N 0,41 |

### b) Polyrotaxan aus dem 6,6',6'',6''',6'''',6'''''-Hexa-Gadolinium-GlyMeDOTA-ester des α-Cyclodextrins und dem PEG-bisamid-derivat aus O,O'-Bis(glycyl)-PEG und N_{α}-Benzyloxycarbonyl-phenylalanin

2,3 g (0,13 mmol) der Titelverbindung aus Beispiel 2a, 8,24 g (13 mmol) der in Beispiel 1f beschriebenen Gadolinium-GlyMeDOTA-säure und 1,34 g (13 mmol) Natriumbromid werden analog der in Beispiel 1h gegebenen Vorschrift umgesetzt und aufgearbeitet. Man erhält 6,67 g (70,2 % d. Th.) farbloses, flockiges Lyophilisat.
H₂O-Gehalt (Karl-Fischer): 5,6 %
Gd-Bestimmung (AAS): 17,7 %

| Elementaranalyse (berechnet auf wasserfreie Substanz): | | | | |
|---|---|---|---|---|
| ber. | C 39,84 | H 5,16 | N 8,60 | Gd 19,13 |
| gef. | C 39,41 | H 5,30 | N 8,84 | Gd 18,80 |

### Beispiel 3

### a) 6,6',6'',6''',6'''',6'''''-Hexa-Gadolinium-GlyMeDOTA-ester des α-Cyclodextrins

20 g (31,76 mmol) der Titelverbindung aus Beispiel 1f, 6.53 g (63,5 mmol) Natriumbromid und 4,41 g (4,54 mmol) α-Cyclodextrin werden in 200 ml Formamid gelöst und auf 10°C gekühlt. Nun gibt man 7,22 g (35 mmol) N,N'-Dicyclohexylcarbodiimid und 0,122 g (1 mmol) 4-Dimethylaminopyridin (DMAP) hinzu und rührt 2 Stunden bei 10°C und 10 Stunden bei Raumtemperatur. Die Lösung wird in eine Mischung aus 1000 ml Aceton/200 ml Diethylether gegossen und eine Stunde bei Raumtemperatur gerührt. Der ausgefallene Niederschlag wird abfiltriert, mit wenig Wasser gelöst und an einer RP-18 Säule chromatographiert (Laufmittel: Gradient aus Acetonitril/Tetrahydrofuran/Wasser).
Ausbeute: 7,59 g (36 % d. Th.) eines farblosen Pulvers
H₂O-Gehalt (Karl-Fischer): 6,8 %

| Elementaranalyse (berechnet auf wasserfreie Substanz): | | | | |
|---|---|---|---|---|
| ber. | C 38,80 | H 4,95 | N 9,05 | Gd 20,32 |
| gef. | C 38,51 | H 5,25 | N 8,76 | Gd 19.88 |

### b) Polyrotaxan aus dem 6,6',6'',6''',6'''',6'''''-Hexa-Gadolinium-GlyMeDOTA-ester des α-Cyclodextrins und dem PEG-bis-ester aus PEG und Gadolinium-GlyMeDOTA-Säure

10,3 g (2,23 mmol) der im Beispiel 3a beschriebenen Titelverbindung werden in 80 ml Wasser gelöst. Nach Zugabe von 340 mg (0,1 mmol) Polyethylenglykol 3400 (Shearwater Polymers, Inc.) wird 15 Minuten bei Raumtemperatur im Ultraschallbad und anschließend über Nacht ohne Beschallung gerührt. Zur Abtrennung von niedermolekularen Bestandteilen wird über eine AMICON®-Ultrafiltrationsmembran YM10 (cut off 10.000 Dalton) filtriert und anschließend gefriergetrocknet. Man erhält 2,6 g farbloses, flockiges Lyophilisat. Der Rückstand wird mit Toluol versetzt und die Suspension dreimal zur Trockne eingedampft. Anschließend wird in 250 ml Formamid gelöst, mit 151 mg (0,24 mmol) Gadolinium-GlyMeDOTA-Säure (Beispiel 1f) versetzt und auf 10°C gekühlt. Nun gibt man 50 mg (0,24 mmol) N,N'-Dicyclohexylcarbodiimid und 12 mg (0,1 mmol) 4-Dimethylaminopyridin (DMAP) hinzu, rührt 2 Stunden bei 10°C und über Nacht bei Raumtemperatur. Die Lösung wird mit 1 l Aceton versetzt, der ausgefallene Niederschlag abfiltriert und erneut über eine YM10 Ultrafiltrationsmembran filtriert. Das Retentat wird eingefroren und gefriergetrocknet. Ausbeute: 2,5 g (45 % d. Th.)
H₂O-Gehalt (Karl-Fischer): 8,0 %
Gd-Bestimmung (AAS): 17,2 %
Aus der Elementaranalyse ergibt sich eine Besetzung von 10 α-Cyclodextrinen/PEG.

| Elementaranalyse (berechnet auf wasserfreie Substanz): | | | | |
|---|---|---|---|---|
| ber. | C 39,77 | H 5,22 | N 8,52 | Gd 19,12 |
| gef. | C 39,71 | H 5,46 | N 8,22 | Gd 19,03 |

### Beispiel 4

### a) 6,6',6",6''',6'''',6''''',6''''''-Hepta-Gadolinium-GlyMeDOTA-ester des β-Cyclodextrins

20 g (31,76 mmol) der Titelverbindung aus Beispiel 1f, 6,53 g (63,5 mmol) Natriumbromid und 4,51 g (3,97 mmol) β-Cyclodextrin werden in 200 ml Formamid gelöst und auf 10°C gekühlt. Man gibt 7,22 g (35 mmol) N,N'-Dicyclohexylcarbodiimid und 0,122 g (1 mmol) 4-Dimethylaminopyridin hinzu und rührt 2 Stunden bei 10°C und 10 Stunden bei Raumtemperatur. Die Lösung wird in eine Mischung aus 1000 ml Aceton/200 ml Diethylether gegossen und eine Stunde bei Raumtemperatur gerührt. Der ausgefallene Niederschlag wird abfiltriert, mit wenig Wasser gelöst und ein einer RP-18 Säule chromatographiert (Laufmittel: Gradient aus Acetonitril/Tetrahydrofuran/Wasser).
Ausbeute: 6,88 g (32 % d. Th.) eines farblosen amorphen Pulvers
Wassergehalt: 7,5 %

| Elementaranalyse (berechnet auf wasserfreie Substanz): | | | | |
|---|---|---|---|---|
| ber. | C 38,80 | H 4,95 | N 9,05 | Gd 20,32 |
| gef. | C 38,47 | H 5,19 | N 8,81 | Gd 19,79 |

### b) Polyrotaxan aus dem 6,6',6'',6''',6'''',6''''',6''''''-Hepta-Gadolinium-GlyMeDOTA-ester des β-Cyclodextrins und dem Polypropylenglykol-bisamid-derivat aus O,O'-Bis(aminopropyl) polypropylenglykol und Gadolinium-GlyMeDOTA-Säure

12,1 g (2,23 mmol) der im Beispiel 4a beschriebenen Titelverbindung werden in 90 ml Wasser gelöst. Nach Zugabe von 200 mg (0,1 mmol) Poly(propylenglykol)-bis(2-aminopropylether) (Sigma) wird 15 Minuten bei Raumtemperatur im Ultraschallbad und anschließend über Nacht ohne Beschallung gerührt. Zur Abtrennung von niedermolekularen Bestandteilen wird über eine AMICON®-Ultrafiltrationsmembran YM10 (cut off 10.000 Dalton) filtriert und anschließend gefriergetrocknet. Man erhält 3,1 g farbloses, flockiges Lyophilisat. Der Rückstand wird mit Toluol versetzt und die Suspension dreimal zur Trockne eingedampft. Gleichzeitig werden 189 mg (0,3 mmol) der in Beispiel 1f beschriebenen Gadolinium-GlyMeDOTA-Säure und 35 mg N-Hydroxysuccinimid in 5 ml Dimethylsulfoxid unter Erwärmen gelöst. Nach Abkühlen auf Raumtemperatur werden 62 mg N,N'-Dicyclohexylcarbodiimid zugesetzt und 60 Minuten gerührt. Zu der so hergestellten N-Hydroxysuccinimidester-Lösung gibt man 3,1 g des oben beschriebenen getrockneten Pseudopolyrotaxans, gelöst in 50 ml DMSO, und 30 mg (0,3 mmol) Triethylamin und rührt über Nacht. Die Lösung wird anschließend mit Aceton gefällt. Der Niederschlag wird abfiltriert, mit Aceton gewaschen und über eine YM10 Ultrafiltrationsmembran filtriert. Das Retentat wird eingefroren und gefriergetrocknet.
Ausbeute: 3,1 g (50 % d. Th.)
H₂O-Gehalt (Karl-Fischer): 6,9 %
Gd-Bestimmung (AAS): 18,2 %
Aus der Elementaranalyse ergibt sich eine Besetzung von 10 β-Cyclodextrinen/Polypropylenglykol.

| Elementaranalyse (berechnet auf wasserfreie Substanz): | | | | |
|---|---|---|---|---|
| ber. | C 39,58 | H 5,14 | N 8,83 | Gd 19.71 |
| gef. | C 39,43 | H 5,02 | N 8,97 | Gd 19,44 |

### Beispiel 5

### Polyrotaxan aus dem 6,6',6'',6''',6'''',6''''',6''''''-Hepta-Gadolinium-GlyMeDOTA-ester des β-Cyclodextrins und dem O,O'-Polypropylenglykol-bis-ester aus Polypropylenglykol und Gadolinium-GlyMeDOTA-Säure

12,1 g (2,23 mmol) der im Beispiel 4a beschriebenen Titelverbindung werden in 90 ml Wasser gelöst. Nach Zugabe von 200 mg (0,1 mmol) Poly(propylenglykol) (Sigma) wird 15 Minuten bei Raumtemperatur im Ultraschallbad und anschließend über Nacht ohne Beschallung gerührt. Zur Abtrennung von niedermolekularen Bestandteilen wird in Wasser über eine AMICON®-Ultrafiltrationsmembran YM10 (cut off 10.000 Dalton) filtriert und anschließend gefriergetrocknet. Man erhält 3,2 g farbloses, flockiges Lyophilisat. Der Rückstand wird mit Toluol versetzt und die Suspension dreimal zur Trockne eingedampft. Anschließend wird in 250 ml Formamid gelöst, mit 151 mg (0,24 mmol) der in Beispiel 1f beschriebenen Gadolinium-GlyMeDOTA-Säure versetzt und auf 10°C gekühlt. Nun gibt man 50 mg (0,24 mmol) N,N'-Dicyclohexylcarbodiimid und 12 mg (0,1 mmol) 4-Dimethylaminopyridin (DMAP) hinzu, rührt 2 Stunden bei 10°C und über Nacht bei Raumtemperatur. Die Lösung wird mit 1l Aceton versetzt, der ausgefallene Niederschlag abfiltriert und erneut über eine YM 10 Ultrafiltrationsmembran filtriert. Das Retentat wird eingefroren und gefriergetrocknet.
Ausbeute: 3,1 g (56,0 % d. Th.)
H₂O-Gehalt (Karl-Fischer): 6,3 %
Gd-Bestimmung (AAS): 18,1 %
Aus der Elementaranalyse ergibt sich eine Besetzung von 9 α-Cyclodextrinen/Polypropylenglykol.

| Elementaranalyse (berechnet auf wasserfreie Substanz): | | | | |
|---|---|---|---|---|
| ber. | C 39,64 | H 5,15 | N 8,76 | Gd 19,67 |
| gef. | C 39,88 | H 5,02 | N 8,96 | Gd 19,14 |

### Beispiel 6

### a) 6,6',6'',6''',6'''',6''''',6''''''-Heptaamino-6,6',6'',6''',6'''',6''''',6''''''-heptadeoxy-β-cyclodextrin-heptahydrochlorid

1,90 g (1 mmol) 6,6',6",6"',6"",6""',6"""-Heptaamino-6,6',6",6"',6"",6""',6"""heptadeoxy-β-cyclodextrin-2,2',2",2'",2"",2""',2""",3,3',3",3'",3"",3""',3"""tetradecaacetat [J. Boger et al., Helv. Chim. Acta 61, 2190-2218 (1978)] werden in Dioxan/Methanol (10:1) gelöst und nach Zugabe von 14 ml (28 mmol) 2 N Natronlauge 2 Stunden bei Raumtemperatur gerührt und anschließend mit verdünnter Salzsäure auf pH 7 gestellt. Die neutralisierte Lösung wird im Vakuum zum Trockne eingedampft, der Rückstand nacheinander mit Chloroform und Wasser gewaschen und wieder zur Trockne eingedampft. Das erhaltene heptakis (6-azido-6-deoxy)-β-CD wird unter Stickstoff in Dioxan/Methanol (5:1) suspendiert, mit 5,25 g (20 mmol) Triphenylphosphin versetzt, die entstandene Lösung eine Stunde bei Raumtemperatur gerührt und anschließend mit konz. Ammoniak versetzt. Nach Rühren über Nacht wird im Vakuum zur Trockne eingedampft, mit Wasser aufgenommen und mit 1 N Salzsäure auf pH 6 gestellt und die Lösung gefriergetrocknet. Man erhält 1,33 g farbloses flockiges Pulver (85 % d. Th.).
H₂O-Gehalt (Karl-Fischer): 11,5 %

| Elementaranalyse (bezogen auf wasserfreie Substanz): | | | | |
|---|---|---|---|---|
| ber. | C 36,47 | H 6,12 | N 7,09 | Cl 17,94 |
| gef. | C 36,77 | H 6,21 | N 6,86 | Cl 17,71 |

### b) Hepta-Amid aus 6,6',6'',6''',6'''',6''''',6''''''-heptaamino-6,6',6'',6''',6'''',6''''',6''''''-heptadeoxy-β-cyclodextrin und DTPA(t-butyl)₄monocarbonsäure

20 g (32,37 mmol) 3,9-Bis-(tert.-butoxycarbonylmethyl)-6-Carboxymethyl-3,6,9-triazaundecan-di-tert.-butylester (hergestellt nach DE 19507822, Schering AG) und 5,56 g (40 mmol) 4-Nitrophenol werden in 200 ml Dimethylformamid gelöst und auf 0°C abgekühlt. Man gibt 7,43 g (36 mmol) N,N'-Dicyclohexylcarbodiimid zu und rührt 2 Stunden bei 0°C. anschließend 12 Stunden bei Raumtemperatur. Zu dieser Lösung gibt man 4,56 g (4,05 mmol) 6,6',6'',6''',6'''',6''''',6''''''-Heptaamino-6,6',6'',6''',6'''',6''''',6''''''-heptadeoxy-β-cyclodextrin und erwärmt 12 Stunden bei 40°C. Man gibt 0,5 ml Wasser zu, rührt 10 Minuten bei 40°C und kühlt auf 0°C ab. Man filtriert vom ausgefallenen Dicyclohexylharnstoff ab und dampft das Filtrat im Vakuum zur Trockne ein. Der Rückstand wird an Kieselgel chromatographiert (Laufmittel: Ethylacetat/Ethanol: 20:1).
Ausbeute: 14,88 g (69 % d. Th.) eines farblosen zähen Öls

| Elementaranalyse: | | | |
|---|---|---|---|
| ber. | C 56,83 | H 8,48 | N 7,36 |
| gef. | C 56,66 | H 8,61 | N 7,21 |

### c) Hepta-Amid aus 6,6',6'',6''',6'''',6''''',6''''''-Heptaamino-6,6',6'',6''',6'''',6''''',6''''''-heptadeoxy-β-cyclodextrin und Gadolinium-DTPA

12 g (2,25 mmol) der Titelverbindung aus Beispiel 6b und 10,8 g (100 mmol) Anisol werden in 200 ml Trifluoressigsäure gelöst. Man dampft im Vakuum zur Trockne ein, nimmt den Rückstand in 800 ml Diethylether auf und rührt eine Stunde bei Raumtemperatur. Der ausgefallene Feststoff wird in 200 ml Wasser gelöst und der pH mit 2 N aqu. Natronlauge auf pH 4 gestellt. Man gibt 5.27 g (15,77 mmol) Gadoliniumacetat zu und rührt 2 Stunden bei 70°C. Man kühlt auf Raumtemperatur und stellt mit 2 N aqu. Natronlauge auf pH 7,2. Die Lösung wird filtriert und das Filtrat in eine Ultrafiltrationszelle eingefüllt (AMICON YM 3, cut off 3 kDa). Man dialysiert mit Wasser (6 Durchläufe); anschließend wird gefriergetrocknet.
Ausbeute: 10,66 g (95 % d. Th.) eines farblosen, amorphen Pulvers
Wassergehalt: 10,3 %

| Elementaranalyse (berechnet auf wasserfreie Substanz): | | | | | |
|---|---|---|---|---|---|
| ber. | C 33,71 | H 3,96 | N 7,86 | Gd 22,06 | Na 3,23 |
| gef. | C 33,51 | H 4,19 | N 7,66 | Gd 21,87 | Na 3,01 |

### d) Polyrotaxan aus dem GdDTPA-heptaamid des 6,6',6'',6''',6'''',6''''',6''''''-heptaamino-6,6',6'',6''',6'''',6''''',6''''''-heptadeoxy-β-cyclodextrins und dem Polypropylenglykol-bisamid-derivat aus O,O'-Bis(aminopropyl)-polypropylenglykol und N_{α}-Benzyloxycarbonyl-phenylalanin

10,8 g (2,23 mmol) der im Beispiel 6c beschriebenen Titelverbindung werden in 100 ml Wasser gelöst. Nach Zugabe von 200 mg (0,1 mmol) Poly(propylenglykol)-bis(2-aminopropylether) (Sigma) wird 15 Minuten bei Raumtemperatur im Ultraschallbad und anschließend über Nacht ohne Beschallung gerührt. Zur Abtrennung von niedermolekularen Bestandteilen wird über eine YM 10-Ultrafiltrationsmembran (Amicon®) filtriert und anschließend gefriergetrocknet. Man erhält 3,9 g farbloses, flockiges Lyophilisat. Der Rückstand wird im Vakuum bei 50°C getrocknet. Anschließend werden 103 mg (0,25 mmol) N_{α}-Benzyloxycarbonyl-tyrosin-N-hydroxysuccinimidester (Bachem), 51 mg Triethylamin und 3,9 g des getrockneten β-Cyclodextrin-pseudopolyrotaxans in 40 ml DMSO gelöst und über Nacht bei Raumtemperatur gerührt. Die Lösung wird anschließend mit Aceton gefällt. Der Niederschlag wird abfiltriert, mit Aceton gewaschen und ultrafiltriert (YM 10). Das Retentat wird gefriergetrocknet.
Ausbeute: 3,4 g (49 % d. Th.)
H₂O-Gehalt (Karl-Fischer): 5,9 %
Gd-Bestimmung (AAS): 20,1 %
Aus der Elementaranalyse ergibt sich eine Besetzung von 13 β-Cyclodextrinen/Polypropylenglykol.

| Elementaranalyse (berechnet auf wasserfreie Substanz): | | | | |
|---|---|---|---|---|
| ber. | C 35,97 | H 4,30 | N 7,88 | Gd 21,88 |
| gef. | C 36,20 | H 4,09 | N 7,99 | Gd 21,21 |

### Beispiel 7

### a) 10-[5-(2-Carboxyphenyl)-2-hydroxy-5-oxo-4-aza-pentyl]-1,4,7-tris(carboxymethyl)-1,4,7,10-tetraazacyclododecan

50 g (144,3 mmol) 1,4,7-Tris(carboxymethyl)-1,4,7,10-tetraazacyclododecan (DO3A) werden in 250 ml Wasser gelöst und der pH-Wert mit 5 N Natronlauge auf pH 13 eingestellt. Dann wird innerhalb einer Stunde eine Lösung aus 38,12 g (187,6 mmol) N(2,3-Epoxypropyl)-phthalimid (Aldrich) in 100 ml Dioxan zugetropft, 24 Stunden bei 50°C gerührt und der pH-Wert durch Zugabe von 5 N Natronlauge bei pH 13 gehalten. Die Lösung wird mit 10%iger Salzsäure auf pH 2 gestellt und im Vakuum zur Trockne eingedampft. Der Rückstand wird in etwas Wasser gelöst und an einer Ionenaustauschersäule (Reillex®= Poly-(4-vinyl)-pyridin (man eluiert mit Wasser) gereinigt. Die Hauptfraktionen werden im Vakuum eingedampft und der Rückstand durch Chromatographie an RP-18 (LiChroPrep®/Laufmittel: Gradient aus Tetrahydrofuran/Methanol/Wasser) endgereinigt. Nach Eindampfen der Hauptfraktionen erhält man 63,57 g (71 % d. Th.) eines amorphen Feststoffes.
Wassergehalt: 8,5 %

| Elementaranalyse (bezogen auf wasserfreie Substanz): | | | |
|---|---|---|---|
| ber. | C 52,90 | H 6,57 | N 12,34 |
| gef. | C 52,65 | H 6,68 | N 12,15 |

### b) 10-(3-Amino-2-hydroxy-propyl)-1,4,7-tris(carboxymethyl)-1,4,7,10-tetraazacyclododecan

50 g (88,1 mmol) der Titelverbindung aus Beispiel 7a werden in 300 ml konz. Salzsäure 24 Stunden unter Rückfluß erhitzt. Man dampft zur Trockne ein. löst den Rückstand in etwas Wasser und reinigt an einer Ionenaustauschersäule (Reillex® = Poly-(4-vinyl)pyridin (man eluiert mit Wasser). Die Hauptfraktionen werden zur Trockne eingedampft Ausbeute: 39,0 g (95 % d. Th.) eines glasigen Feststoffes
Wassergehalt: 10,3 %

| Elementaranalyse (bezogen auf wasserfreie Substanz): | | | |
|---|---|---|---|
| ber. | C 48,68 | H 7,93 | N 16,70 |
| gef. | C 48,47 | H 8,09 | N 16,55 |

### c) Gadolinium-Komplex des 10-(3-Amino-2-hydroxy-propyl)-1,4,7-tris(carboxymethyl)-1,4,7,10-tetraazacyclododecans

38 g (90,6 mmol) der Titelverbindung aus Beispiel 7b werden in 300 ml Wasser gelöst und 16,42 g (45,3 mmol) Gadoliniumoxid zugesetzt. Man erwärmt 3 Stunden auf 90°C. Die abgekühlte Lösung wird mit je 5 ml saurem Ionenaustauscher (IR-120/H⁺-Form) und 5 ml basischem Austauscher (IRA-410/OH⁻-Form) eine Stunde bei Raumtemperatur gerührt. Man filtriert vom Austauscher ab. Gefriertrocknung des Filtrats liefert 57,23 g (98 % d.Th.) eines amorphen Feststoffes.
Wassergehalt: 11,3 %

| Elementaranalyse (bezogen auf wasserfreie Substanz): | | | | |
|---|---|---|---|---|
| ber. | C 35,59 | H 5,27 | Gd 27,41 | N 12,21 |
| gef. | C 35,32 | H 5,38 | Gd 27,20 | N 12,31 |

### d) Gadolinium-Komplex des 10-[7-(4-Nitrophenyl)-2-hydroxy-5-oxo-7-(carboxymethyl)-4-azaheptyl]-1,4,7-tris(carboxymethyl)-1,4,7,10-tetraazacyclododecans

Zu 20 g (34,86 mmol) der Titelverbindung aus Beispiel 7c in 200 ml Dimethylformamid/20 ml Triethylamin gibt man 9,84 g (41,8 mmol) 3-(4-Nitrophenyl)-glutarsäureanhydrid (Journal of Org. Chem., Vol. 26, 3856 (1961)) und rührt über Nacht bei Raumtemperatur. Man dampft im Vakuum zur Trockne ein. Der Rückstand wird aus Isopropanol/Essigsäure 95:5 umkristallisiert.
Ausbeute: 27,46 g (94 % d. Th.) eines gelblichen Feststoffes
Wassergehalt: 3,4 %

| Elementaranalyse (bezogen auf wasserfreie Substanz): | | | | |
|---|---|---|---|---|
| ber. | C 41,58 | H 4,86 | Gd 19,44 | N 10,39 |
| gef. | C 41,38 | H 4,97 | Gd 19,28 | N 10,17 |

### e) Gadolinium-Komplex des 10-[7-(4-Aminophenyl)-2-hydroxy-5-oxo-7-(carboxymethyl)-4-aza-heptyl]-1,4,7-tris(carboxymethyl)-1,4,7,10-tetraazacyclododecans

25 g (30,9 mmol) der Titelverbindung aus Beispiel 7d werden in 250 ml Methanol gelöst und 5 g Palladium-Katalysator (10 % Pd auf C) zugegeben. Man hydriert über Nacht bei Raumtemperatur. Der Katalysator wird abfiltriert und das Filtrat im Vakuum zur Trockne eingedampft.
Ausbeute: 24,07 g (97 % d. Th.) eines cremefarbenen Feststoffes
Wassergehalt: 3,0 %

| Elementaranalyse (bezogen auf wasserfreie Substanz): | | | | |
|---|---|---|---|---|
| ber. | C 43,18 | H 5,31 | Gd 20,19 | N 10,79 |
| gef. | C 43,27 | H 5,48 | Gd 20,02 | N 10,61 |

### f) Gadolinium-Komplex des 10-[7-(4-Isothiocyanatophenyl)-2-hydroxy-5-oxo-7-(carboxymethyl)-4-aza-heptyl]-1,4,7-tris(carboxymethyl)-1,4,7,10-tetraazacyclododecans

15 g (19,26 mmol) der Titelverbindung aus Beispiel 7e werden in 100 ml Wasser gelöst und 6,64 g (57,8 mmol) Thiophosgen in 50 ml Chloroform zugegeben. Man rührt 1 Stunde bei 50°C. Man kühlt auf Raumtemperatur, trennt die organische Phase ab und schüttelt die wässrige Phase 2 mal mit 100 ml Chloroform aus. Die wässrige Phase wird zur Trockne eingedampft und der Rückstand in 100 ml Isopropanol bei Raumtemperatur ausgerührt. Der Feststoff wird abfiltriert und mit Ether gewaschen. Nach Trocknen über Nacht im Vakuum (40°C) erhält man 15,9 g (98 % d. Th.) eines cremefarbenen Feststoffes.
Wassergehalt: 3,5 %

| Elementaranalyse (bezogen auf wasserfreie Substanz): | | | | | |
|---|---|---|---|---|---|
| ber. | C 42,43 | H 4,79 | Gd 19,15 | N 10,24 | S 3,91 |
| gef. | C 42,23 | H 4,90 | Gd 19,01 | N 10,05 | S 3,96 |

### g) 6,6',6",6"',6"",6""'-Hexakis(N-Fluorenylmethoxycarbonyl-alanyl)-α-cyclodextrin

2,08 g (2 mmol; Wassergehalt 7 %) α-Cyclodextrin werden in Dimethylformamid gelöst, mit Benzol versetzt und azeotrop entwässert. Es wird anschließend auf 0°C gekühlt, 1,32 ml (12 mmol) N-Methylmorpholin zugegeben und nach Zugabe von 4,05 g (12 mmol) N-Fluorenylmethoxycarbonyl-alanin-N-carbonsäureanhydrid, Fmoc-Ala-NCA (Propeptide,SNPE GmbH, Frankfurt/M.) wird über Nacht bei Raumtemperatur gerührt. Die Lösung wird dann im Vakuum eingedampft, der Rückstand mit Wasser gewaschen und schließlich aus Essigester umkristallisiert.
Ausbeute: 5,08 g (93 % d. Theorie)

| Elementaranalyse (bezogen auf wasserfreie Substanz): | | | |
|---|---|---|---|
| ber. | C 63,29 | H 5,53 | N 3,08 |
| gef. | C 63,14 | H 5,70 | N 2,98 |

### h) Hexa-Thioharnstoff-Konjugat aus Hexakis-(O-alanyl)-α-CD mit dem Gadolinium-Komplex des 10-[7-(4-Isothiocyanatophenyl)-2-hydroxy-5-oxo-7-(carboxymethyl)-4-azaheptyl]-1,4,7-tris(carboxymethyl)-1,4,7,10-tetraazacyclododecans

2,78 g (1 mmol) des im vorstehenden Beispiel 7g beschriebenen Hexakis-[O-(Fmoc-alanyl)]-α-CD's werden in Dimethylformamid gelöst und mit 4 g 4-Dimethylaminopyridin versetzt. Nach 15 Minuten Rühren bei Raumtemperatur werden 5,31 g (6,3 mmol) des in Beispiel 7f beschriebenen Isothiocyanats zugegeben und über Nacht gerührt. Die Lösung wird anschließend im Vakuum eingeengt, mit Wasser aufgenommen, auf pH 7 eingestellt und über eine AMICON® Ultrafiltrationsmembran YM 1 von niedermolekularen Anteilen befreit. Nach beendeter Ultrafiltration wird mit verdünnter Natronlauge nochmals auf pH 7 eingestellt, das Retentat eingefroren und gefriergetrocknet. Man erhält 6,78 g (97 % d. Theorie) leicht gelbliches flockiges Pulver. Eine analytische Probe-Anfärbung mit Ninhydrin zeigt, daß keine freien Aminogruppen mehr in dem Thio-Harnstoff-Konjugat vorhanden sind.
Wassergehalt (Karl-Fischer): 8,3 %
Gd-Bestimmung (AAS): 13,2 %

| Elementaranalyse (bezogen auf wasserfreie Substanz): | | | | | | |
|---|---|---|---|---|---|---|
| ber. | C 42,41 | H 4,96 | Gd 14,61 | N 9,11 | Na 2,14 | S 2,98 |
| gef. | C 42,25 | H 5,10 | Gd 14,26 | N 9,01 | Na 1,79 | S 2,66 |

### i) Polyrotaxan aus dem im vorstehenden Beispiel 7h beschriebenen Hexa-Thioharnstoff-Konjugat des α-Cyclodextrins und dem N,N'-Bis-Thioharnstoff-Konjugat aus O,O'-Bis(aminoethyl)-PEG und dem Gadolinium-Komplex des 10-[7-(4-Isothiocyanatophenyl)-2-hydroxy-5-oxo-7-(carboxymethyl)-4-azaheptyl]-1,4,7-tris(carboxymethyl)-1,4,7,10-tetraazacyclododecans

22,60 g (3,5 mmol) des in Beispiel 7h beschriebenen Hexa-Thioharnstoff-Konjugats des α-Cyclodextrins werden in 200 ml Wasser gelöst. Nach Zugabe von 337 mg (0,1 mmol) O,O'-Bis(aminoethyl)-PEG (Sigma) wird 30 Minuten um Ultraschallbad und über Nacht ohne Beschallung bei Raumtemperatur gerührt. Zur Abtrennung der nicht aufgefädelten Komponenten wird ultrafiltriert (YM 10) und anschließend gefriergetrocknet. Man erhält 7,2 g leicht gelbliches Pulver. Der Rückstand wird erneut in Wasser aufgenommen, mit 211 mg (0,25 mmol) des in Beispiel 7f beschriebenen Isothiocyanats versetzt und über Nacht gerührt. Die Lösung wird anschließend auf pH 7 eingestellt, ultrafiltriert (YM 30, cut off 30 kDa) und das Retentat gefriergetrocknet. Man erhält 6,9 g (45,9 % d. Th.) leicht gelbes flockiges Pulver.
H₂O-Gehalt (Karl-Fischer): 10,2 %
Gd-Bestimmung (AAS): 12,8 %
Aus der Elementaranalyse ergibt sich eine Besetzung von 20 α-Cyclodextrinen/PEG

| Elementaranalyse (berechnet auf wasserfreie Substanz): | | | | | | |
|---|---|---|---|---|---|---|
| ber. | C 42,69 | H 5,07 | N 8,91 | S 2,92 | Gd 14,30 | Na 2,09 |
| gef. | C 42,20 | H 5,31 | N 8,67 | S 2,34 | Gd 14,56 | Na 1,80 |

### Beispiel 8

### a) Hexa-Amid aus 6,6',6'',6''',6'''',6'''''-Hexaamino, 6,6',6'',6''',6'''',6'''''-hexadeoxy-α -cyclodextrin und Gadolinium-GlyMeDOTA-Säure

20 g (31,76 mmol) der Titelverbindung aus Beispiel 1f, 2,69 g (63,5 mmol) Lithiumchlorid und 5,56 g (40 mmol) 4-Nitrophenol werden in 250 ml Dimethylsulfoxid bei 60°C gelöst. Man kühlt auf 16°C ab und gibt 7,22 g (35 mmol) N,N'-Dicyclohexylcarbodiimid hinzu und rührt 12 Stunden bei Raumtemperatur. Zu dieser Lösung gibt man 4,39 g (4,54 mmol) 6,6',6'',6''',6'''',6'''''-Hexaamino-6,6',6'',6''',6'''',6'''''-hexadeoxy-α-cyclodextrin [J. Boger et al., Helv. Chim. Acta 61, 2190-2218 (1978)] und erwärmt 12 Stunden bei 60°C. Man kühlt auf Raumtemperatur ab und gießt die Lösung in eine Michung aus 800 ml Aceton und 200 ml Diethylether und und rührt eine Stunde bei Raumtemperatur. Der Niederschlag wird abfiltriert und in 200 ml Wasser gelöst. Der dabei ausgefallene N,N'-Dicyclohexylharnstoff wird abfiltriert und das Filtrat in eine Ultrafiltrationszelle (AMICON, YM 3, cut off 3 kDa) gebracht. Man dialisiert mit Wasser (6 Durchläufe). Anschließend wird gefriergetrocknet.
Ausbeute: 15,58 g (74 % d. Th.) eines farblosen amorphen Pulvers
H₂O-Gehalt (Karl-Fischer): 6,9 %

| Elementaranalyse (berechnet auf wasserfreie Substanz): | | | | |
|---|---|---|---|---|
| ber. | C 38,85 | H 5,09 | N 10,87 | Gd 20,35 |
| gef. | C 38,51 | H 5,31 | N 10,61 | Gd 20,11 |

### b) Polyrotaxan aus dem im vorstehenden Beispiel 8a beschriebenen Hexaamid des α-Cyclodextrins und dem PEG-bisamid-derivat aus O,O'-Bis-(glycyl)-PEG und Gadolinium-GlyMeDOTA-Säure

10,3 g (2,23 mmol) der im Beispiel 8a beschriebenen Titelverbindung werden in 80 ml Wasser gelöst. Nach Zugabe von 340 mg (0,1 mmol) O,O'-Bis(glycyl)-PEG (Shearwater Polymers, Inc.) wird 15 Minuten bei Raumtemperatur im Ultraschallbad und anschließend über Nacht ohne Beschallung gerührt. Zur Abtrennung von niedermolekularen Bestandteilen wird über eine AMICON®-Ultrafiltrationsmembran YM10 (cut off 10.000 Dalton) filtriert und anschließend gefriergetrocknet. Man erhält 2,9 g farbloses, flockiges Lyophilisat. Der Rückstand wird mit Toluol versetzt und die Suspension dreimal zur Trockne eingedampft. Gleichzeitig werden 189 mg (0,3 mmol) der in Beispiel 1f beschriebenen Gadolinium-GlyMeDOTA-Säure und 35 mg N-Hydroxysuccinimid in 5 ml Dimethylsulfoxid unter Erwärmen gelöst. Nach Abkühlen auf Raumtemperatur werden 62 mg N,N'-Dicyclohexylcarbodiimid zugesetzt und 60 Minuten gerührt. Zu der so hergestellten N-Hydroxysuccinimidester-Lösung gibt man 2,9 g des oben beschriebenen getrockneten Pseudopolyrotaxans, gelöst in 50 ml DMSO, und 30 mg (0,3 mmol) Triethylamin und rührt über Nacht. Die Lösung wird anschließend mit Aceton gefällt. Der Niederschlag wird abfiltriert, mit Aceton gewaschen und über eine YM 10 Ultrafiltrationsmembran filtriert. Das Retentat wird eingefroren und gefriergetrocknet.
Ausbeute: 3,0 g (51 % d. Th.)
H₂O-Gehalt (Karl-Fischer): 5,0 %
Gd-Bestimmung (AAS): 18,4 %
Aus der Elementaranalyse ergibt sich eine Besetzung von 11 α-Cyclodextrinen/PEG.

| Elementaranalyse (berechnet auf wasserfreie Substanz): | | | | |
|---|---|---|---|---|
| ber. | C 39,76 | H 5,32 | N 10,26 | Gd 19,19 |
| gef. | C 39,39 | H 5,47 | N 10,60 | Gd 18,74 |

### Beispiel 9

### Polyrotaxan aus dem α-Cyclodextrin-hexaamid, gebildet aus 6,6',6'',6''',6'''',6'''''hexaamino- 6,6',6'',6''',6'''',6'''''-hexadeoxy-α-cyclodextrin und Gadolinium-GlyMeDOTA-Säure, und dem PEG-bisamid-derivat aus O,O'-Bis-(aminoethyl)-PEG und Gadolinium-GlyMeDOTA-Säure im Eintopfverfahren

4,31 g (4,46 mmol) 6,6',6'',6''',6'''',6'''''-hexaamino-6,6',6'',6''',6'''',6'''''-hexadeoxy-α-cyclodextrin werden in 50 ml Wasser gelöst. Nach Zugabe von 340 mg (0,1 mmol) O,O'-Bis(aminoethyl)-PEG (Sigma) wird 30 Minuten bei Raumtemperatur gerührt, die Suspension anschließend noch 3 Minuten im Ultraschallbad behandelt und über Nacht gerührt. Der Niederschlag wird abgesaugt, mit wenig Wasser gewaschen und bei 50°C im Vakuum getrocknet. Man erhält 1,3 g farbloses Pulver. Gleichzeitig werden 6,29 g (10 mmol) der in Beispiel 1f beschriebenen Gadolinium-GlyMeDOTA-Säure und 1,2 g N-Hydroxysuccinimid in 75 ml Dimethylsulfoxid unter Erwärmen gelöst. Nach Abkühlen auf Raumtemperatur werden 2,1 g N,N'-Dicyclohexylcarbodiimid zugesetzt und 60 Minuten gerührt. Zu der so hergestellten N-Hydroxysuccinimidester-Lösung gibt man 1,3 g des oben beschriebenen getrockneten α-Cyclodextrin-pseudopolyrotaxans, gelöst in 25 ml DMSO, und 1,01 g (10 mmol) Triethylamin und rührt über Nacht. Die Lösung wird anschließend mit Diethylether gefällt. Der Niederschlag wird abfiltriert, mit Diethylether gewaschen und an einer RP-18-Säule chromatographiert (Laufmittel: Gradient aus Acetonitril/Tetrahydrofuran/Wasser). Ausbeute: 2,2 g (41,5 % d. Th.) eines farblosen amorphen Pulvers
H₂O-Gehalt (Karl-Fischer): 4,0 %
Gd-Bestimmung (AAS): 18,4 %
Aus der Elementaranalyse ergibt sich eine Besetzung von 10 α-Cyclodextrinen/PEG.

| Elementaranalyse (berechnet auf wasserfreie Substanz): | | | | |
|---|---|---|---|---|
| ber. | C 39,83 | H 5,34 | N 10,23 | Gd 19,13 |
| gef. | C 39,80 | H 5,62 | N 10,03 | Gd 18,79 |

### Beispiel 10

### a) Hexaamid-Derivat der 2,4,6-Trijod-3-N-(2-hydroxyethyl)-5-(hydroxy)acetamidoisophthalsäure mit 6,6',6",6"',6'''',6'''''-Hexaamino-6,6',6",6"',6'''',6'''''-hexadeoxy-α-cyclodextrin

Zu 1,26 g (1 mmol) 6,6',6",6"',6'''',6'''''-Hexamino-6,6',6",6"',6'''',6'''''-hexadeoxy-α-cyclodextrin-hexahydrochlorid [J. Boger, RJ. Corcorcan und J.-M. Lehn, Helv. Chim. Acta 61, 2190-2218 (1978)] in 40 ml N,N-Dimethylacetamid gibt man 2,02 g (20 mmol) Triethylamin und 5,03 g (6,60 mmol) des Säurechlorids der 2,4,6-Trijod-3-N-(2-acetoxyethyl)-5-acetoxy-acetamido-isophtalsäure [Guerbet S.A., WO 93/10824]. Man rührt 24 Stunden bei Raumtemperatur. Es wird im Vakuum zur Trockne eingedampft, der Rückstand in 200 ml Methylenchlorid gelöst und 2 mal mit je 100 ml 5 %iger wäßriger Salzsäure und 100 ml 5 %iger Natriumcarbonatlösung gewaschen. Die organische Phase wird im Vakuum zur Trockne eingedampft, der Rückstand in 200 ml Methanol gelöst und bei 0°C Ammoniak (Gas) bis zur Sättigung eingeleitet. Man rührt 6 Stunden bei 0°C, anschließend 15 Stunden bei 40°C. Man dampft zur Trockne ein und chromatographiert den Rückstand an einer RP-18 Säule (Laufmittel: Gradient aus Wasser/ Acetonitril/n-Propanol).
Ausbeute: 4,1 g (85 % d. Th.) eines farblosen Feststoffes
Wassergehalt: 1,2 %

| Elementaranalyse (bezogen auf wasserfreie Substanz): | | | | |
|---|---|---|---|---|
| ber. | C 26,92 | H 2,51 | N 5,23 | J 47,41 |
| gef. | C 26,71 | H 2,70 | N 5,05 | J 47,19 |

### b) Polyrotaxan aus dem Hexaamid-Derivat der 2,4,6-Trijod-3-N-(2-hydroxyethyl)-5-(hydroxy)acetamido-isophthalsäure mit 6,6',6",6"',6'''',6'''''-Hexaamino-6,6',6",6"',6'''',6'''''-hexadeoxy-α-cyclodextrin und dem PEG-bisamid-derivat aus O,O'-Bis(glycyl)-PEG und N_{α}-Benzyloxycarbonyl-phenylalanin

20,57 g (4,47 mmol) des in Beispiel 10 a beschriebenen jodhaltigen α-Cyclodextrins werden in 200 ml Wasser gelöst. Nach Zugabe von 350 mg (0,1 mmol) O,O'-Bis(glycyl)-PEG (Shearwater Polymers, Inc.) wird 30 Minuten bei Raumtemperatur gerührt, die Suspension anschließend noch 3 Minuten im Ultraschallbad behandelt und über Nacht gerührt. Der Niederschlag wird abgesaugt, mit wenig Wasser gewaschen und bei 50°C im Vakuum getrocknet. Man erhält 4,1 g farbloses Pulver. Anschließend werden 87 mg (0,22 mmol) N_{α}-Benzyloxycarbonyl-phenylalanin-N-hydroxysuccinimidester (Bachem), 45 mg Triethylamin und 4,1 g des getrockneten α-Cyclodextrin-pseudopolyrotaxans in 50 ml DMSO gelöst und über Nacht gerührt. Die Lösung wird anschließend mit 500 ml Diethylether versetzt. Der Niederschlag wird abfiltriert, mit Diethylether gewaschen und an einer RP-18-Säule chromatographiert (Laufmittel: Gradient aus Acetonitril/Tetrahydrofuran/Wasser).
Ausbeute: 3,69 g (41,5 % D. Th.) eines farblosen amorphen Pulvers
H₂O-Gehalt (Karl-Fischer): 6,0 %

Aus der Elementaranalyse ergibt sich eine Besetzung von 12 α-Cyclodextrinen/PEG.

| Elementaranalyse (berechnet auf wasserfreie Substanz): | | | | |
|---|---|---|---|---|
| ber. | C 28,85 | H 2,90 | N 4,98 | 144,31 |
| gef. | C 29,04 | H 2,78 | N 5,12 | I 44,11 |

## Patentansprüche

1. Polyrotaxane enthaltend als bildgebende Komponente für die MRT- und Röntgendiagnostik Metallkomplexe oder jodhaltige Benzolderivate.

2. Polyrotaxane der Formel I 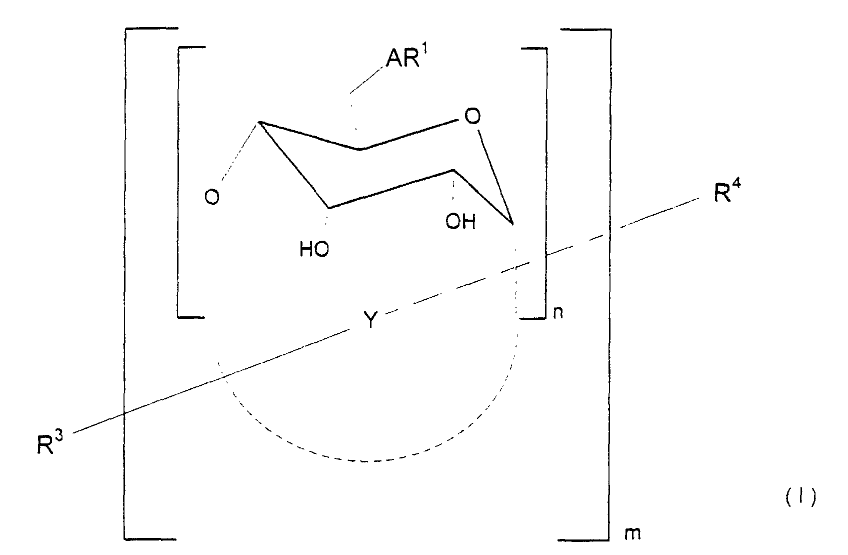 worin
n die Zahlen 6,7 oder 8,
m die Zahlen 2 bis 50,
A ein Sauerstoffatom oder die Gruppe -XNH-,
worin
X eine direkte Bindung oder den Rest -O-(CO)ₓ-CHR-(CH₂)_{y}- mit x in der Bedeutung der Zahlen 0 oder 1 und
y in der Bedeutung der Zahlen 0 bis 10
bedeutet,
R¹ die kontrastgebenden Reste II, III, IV, V, VI, VII, VIII, IX oder X 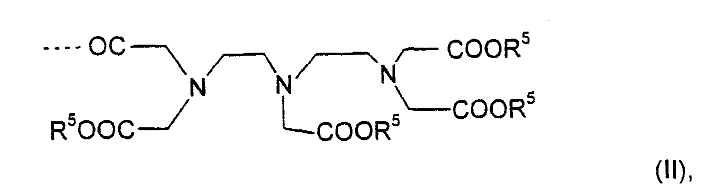 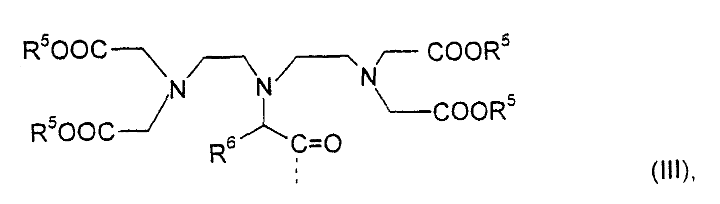 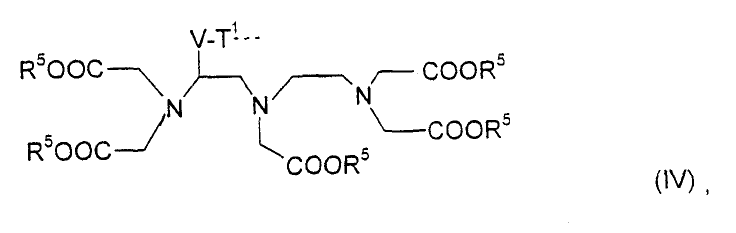 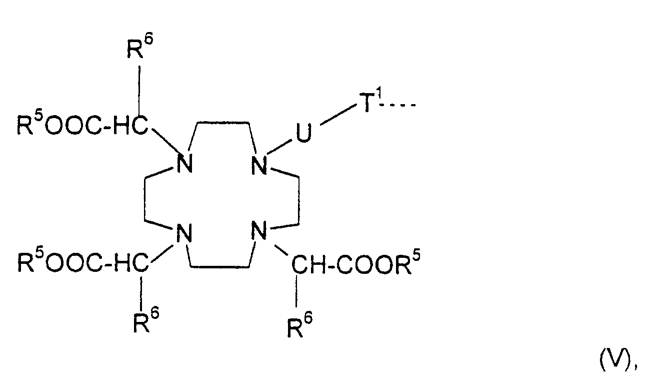 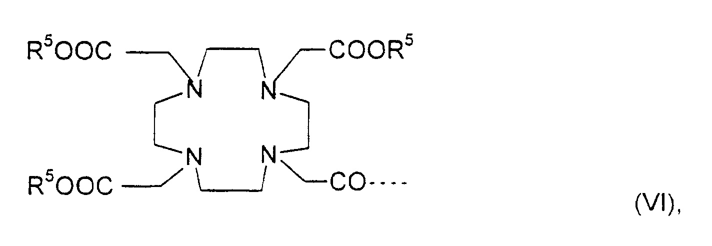 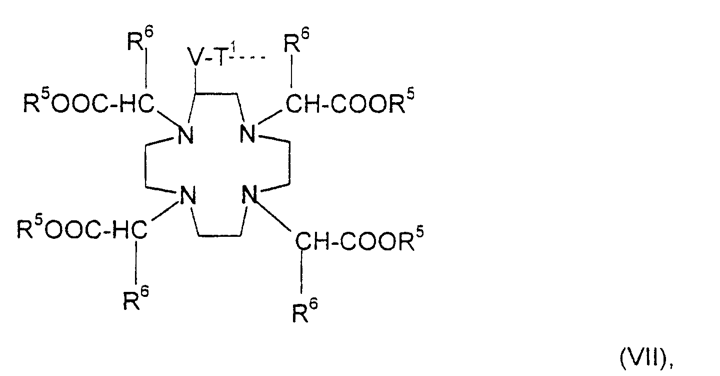 worin
R⁵ unabhängig voneinander ein Wasserstoffatom oder ein Metallionenäquivalent der Elemente der Ordnungszahlen 20-32, 37 - 39, 42 - 44, 49 oder 57 -83,
R⁶ ein Wasserstoffatom, einen geradkettigen oder verzweigten C₁-C₇-Alkylrest, einen Phenyl- oder Benzylrest,
V eine -CH₂-(CH₂)ₒ-(O)ₚ 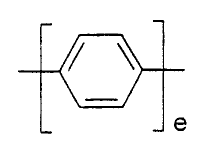 gruppe mit
o in der Bedeutung der Zahlen 0 bis 10,
p und e jeweils in der Bedeutung der Ziffern 0 oder 1, mit der Maßgabe, daß p nur dann für die Ziffer 1 steht, wenn e die Ziffer 1 bedeutet,
T¹ eine -NHCS- oder -CO-gruppe,
U eine -CHR⁷-CONR^{7'}-M¹- oder -CH₂-CH(OH)-M²-gruppe mit R⁷ und R^{7'} unabhängig voneinander in der Bedeutung von R⁶ oder der Gruppe -CH₂-(CH₂)ₒ-COOH und M¹ und M² jeweils in der Bedeutung eines Phenylenrestes oder einer geradkettigen, verzweigten, gesättigten oder ungesättigten C₁-C₂₀-Alkylenkette, die gegebenenfalls substituiert ist durch 1-5 (CH₂)ₒ-COOH, 1-5 OR⁶-reste oder 1-8 Sauerstoffatome, 1-2-NH-, 1-2-C(=NH)-, 1-5-CONR⁷, 1-5-NR⁷CO-, 1-2 Phenylen- oder 1-2 Phenylenoxygruppen enthält, bedeuten, mit der Maßgabe, daß mindestens zwei der Reste R⁵ für Metallionenäquivalente der Elemente der oben genannten Ordnungszahlen stehen sowie gegebenenfalls Kationen anorganischer und/oder organischer Basen, Aminosäuren oder Aminosäureamide, 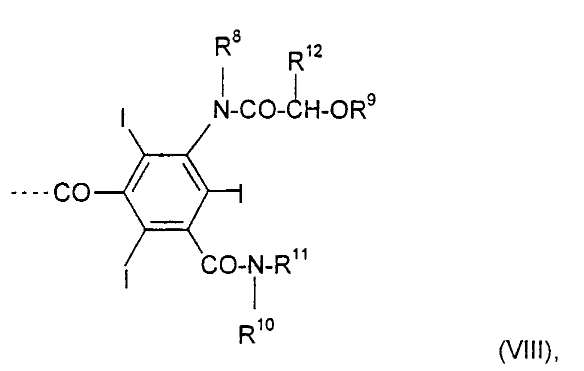 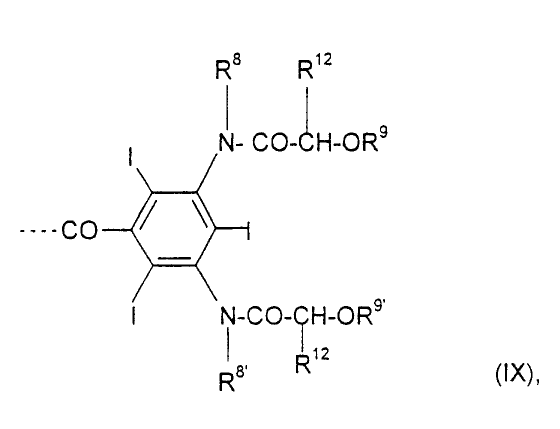 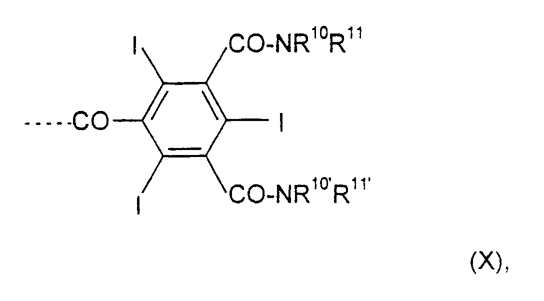 mit
R⁸, R^{8'}, R¹⁰, R^{10'}, R¹¹, R^{11'}, die gleich oder verschieden sein können, in der Bedeutung Wasserstoff oder geradkettiges Alkyl mit 2-6 C-Atomen oder verzweigtkettiges Alkyl mit 3-6 C-Atomen, wobei beide Alkylreste mit 1-5 OH Gruppen substituiert sein können,
R⁹, R^{9'}, R¹², die gleich oder verschieden sein können, in der Bedeutung Wasserstoff oder Methyl und
Y die Reste -W -(CH₂)₂[O-CH₂-CH₂]_{q}-W, 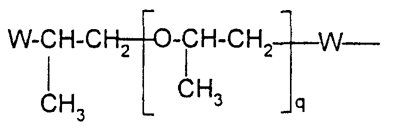 worin q die Zahlen 8 bis 200, W die NH-gruppe oder das O-Atom bedeuten, und
R³ und R⁴ unabhängig voneinander einen Substituenten mit einem Durchmesser von mindestens 0,6 nm bedeuten.

3. Polyrotaxane der Formel I gemäß Anspruch 2, **dadurch gekennzeichnet, daß** die für R³ und R⁴ stehenden Gruppen als Ester, Amid, Ether, Thioester, Thioamid oder Carbonat an Y gebunden sind.

4. Polyrotaxane der Formel I gemäß Anspruch 2 und 3, **dadurch gekennzeichnet, daß** R³ und R⁴ unabhängig voneinander für R¹ oder eine 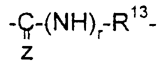 gruppe mit Z in der Bedeutung eines Sauerstoff- oder Schwefelatoms und r in der Bedeutung der Zahlen 0 oder 1, wobei R¹³ für eine gesättigte, ungesättigte, geradkettige oder verzweigte C₁-C₃₀-Alkylkette steht, die unterbrochen sein kann durch 1-3 -NH-CO-O-, -O-OC-NH-, -NHCO-, OCNH-, NHCS-, -SCNH-, -NH-CS-NH-, -NH-CO-NH-, -CO-O-, -O-OC-, NR⁶-, -CO-, -SO₂-, -SO-, 1-2 Phenylen-, Phenylenoxy-, Cyclohexyliden-gruppen, 1-3 Sauerstoff-, Schwefelatome und/oder substituiert sein kann durch 1-5 -OH-, -OCH₃-, 1-3 -CH₂OH-, -NHCOR⁶-, -CONHR⁶-, -COOH-, -(CH₂)₁₋₅-COOH-, -CH₂-C₆H₄-OH-, -CH₂-C₆H₅-, -C₆H₅-, Naphthyl-, Pyridyl-, Cyclohexyl-, Thiophenyl-gruppen, wobei die gegebenenfalls vorhandenen aromatischen Gruppen gegebenenfalls 1-3 Chlor-, Brom-, CH₃-, COOH-, CH₂OH-, OCH₃-Substituenten enthalten können.

5. Pharmazeutische Mittel enthaltend mindestens einen Polyrotaxan-Komplex gemäß allgemeiner Formel I von Anspruch 2, gegebenenfalls mit den in der Galenik üblichen Zusätzen.

6. Verwendung von mindestens einem Polyrotaxan-Komplex gemäß allgemeiner Formel I von Anspruch 2 für die Herstellung von Mitteln für die NMR- oder Röntgendiagnostik.

7. Verwendung von mindestens einem Polyrotaxan-Komplex gemäß allgemeiner Formel I von Anspruch 2 für die Herstellung von Mitteln für die Angiographie.

8. Verwendung von mindestens einem Polyrotaxan-Komplex gemäß allgemeiner Formel I von Anspruch 2 für die Herstellung von Mitteln für die Lymphographie.

9. Verfahren zur Herstellung von Polyrotaxan-Komplexen gemäß allgemeiner Formel I von Anspruch 2, **dadurch gekennzeichnet, daß** man Verbindungen der allgemeinen Formel II 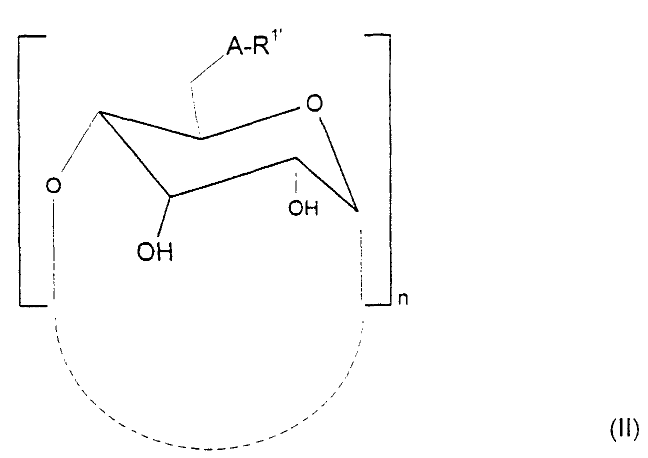 worin
A und n die oben angegebenen Bedeutungen haben und
R^{1'} für ein Wasserstoffatom oder den Rest II', III', IV', V', VI', VII', VIII', IX' oder X', worin diese die für II - X angegebene Bedeutung haben, jedoch die in ihnen vorhandenen Reste R⁵ für Wasserstoff oder Säureschutzgruppen stehen und gegebenenfalls vorhandene Hydroxygruppen gegebenenfalls in geschützter Form vorliegen, steht
mit Verbindungen der allgemeinen Formel XI
H-Y-H (XI)
zu Verbindungen der allgemeinen Formel XII umsetzt 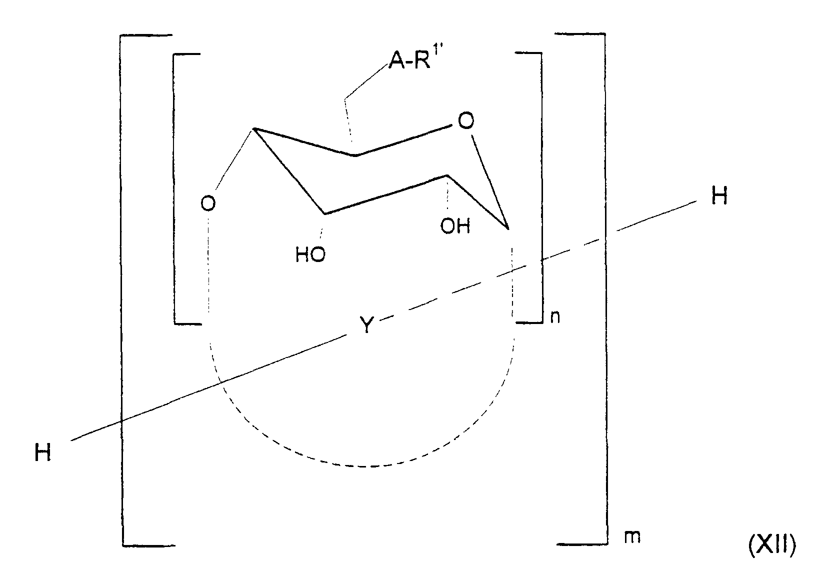 und diese mit einer Verbindung R^{3'}-Fg und/oder R^{4'}-Fg umsetzt,
worin R^{3'} und R^{4'} die für R³ und R⁴ angegebene Bedeutung haben, jedoch die in R¹ stehenden Reste R⁵ für Wasserstoff, Säureschutzgruppen oder ein Metallionenäquivalent der Elemente der genannten Ordnungszahlen und Fg für Anhydrid, ―Cl, ―F, ―OH, ―N₃, 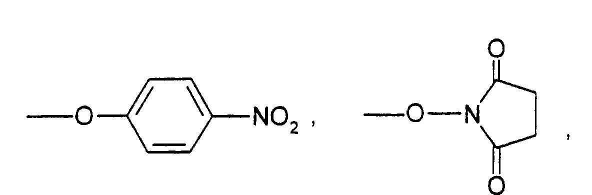 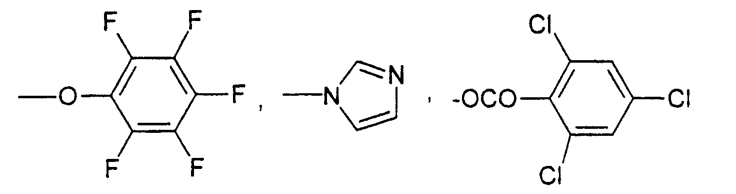 steht,
wobei im Falle, daß R³ und/oder R⁴ in (I) für einen Rest 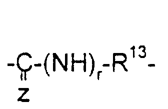 steht, eine Verbindung der allgemeinen Formel R³-N=C=Z bzw. R⁴-N=C=Z eingesetzt wird sowie im Falle, daß in IV, V und VII T¹ für eine NHCS-Gruppe steht, eine Verbindung der allgemeinen Formel IV", V" oder VII' 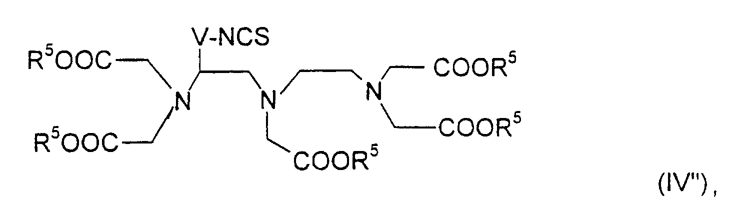 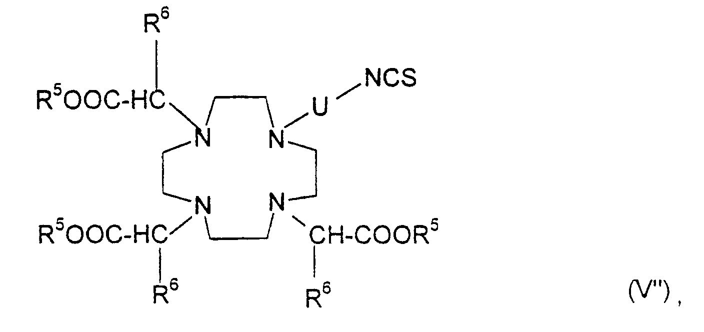 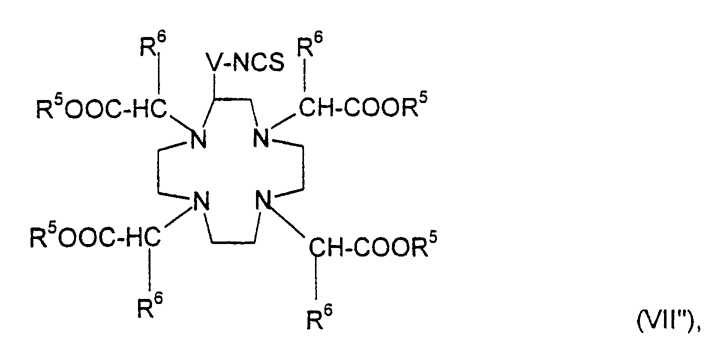 eingesetzt wird, die so erhaltene Verbindung der allgemeinen Formel XIII 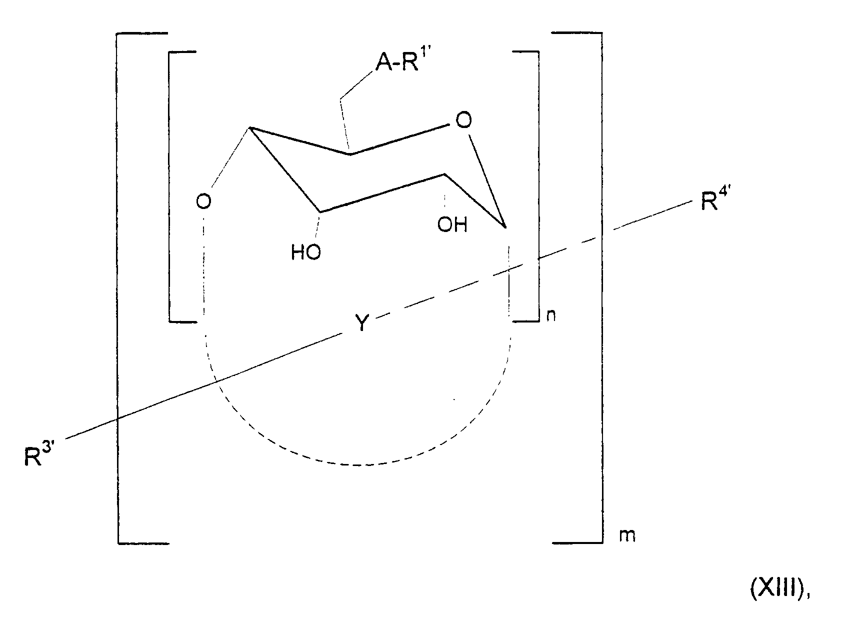 falls R^{1'} die Bedeutung von Wasserstoff hat, mit einer Verbindung R^{1''}-Fg, worin R^{1''} die für R¹ angegebene Bedeutung hat, jedoch die in ihnen vorhandenen Reste R⁵ für Wasserstoff, Säureschutzgruppen oder ein Metallionenäquivalent der Elemente der genannten Ordnungszahlen stehen, bzw. mit Verbindungen der allgemeinen Formel IV'', V'' oder VII'' umsetzt und anschließend, für den Fall, daß R⁵ in XIII nicht für die o.g. Metallionenäquivalente steht, gegebenenfalls vorhandene Säureschutzgruppen abspaltet, die gewünschten Metallionen einführt und anschließend, falls gewünscht, vorhandene acide Wasserstoffatome durch Kationen anorganischer und/oder organischer Basen, Aminosäuren oder Aminosäureamide substituiert.

## Claims

1. Polyrotaxanes that contain metal complexes or iodine-containing benzene derivatives as imaging components for MRT diagnosis and x-ray diagnosis.

2. Polyrotaxanes of formula I 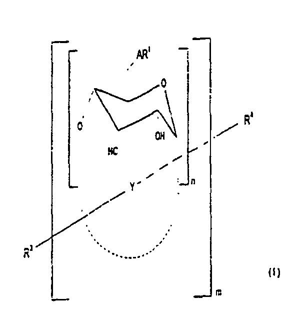 in which n means the numbers 6, 7 or 8,
m means the numbers 2 to 50,
A means an oxygen atom or the group -XNH-, in which
X means a direct bond or the radical -O-(CO)ₓ-CHR-(CH₂)_{y} - with
x meaning numbers 0 or 1 and
y meaning numbers 0 to 10,
R¹ means contrast-imparting radicals II, III, IV, V, VI, VII, VIII, IX or X 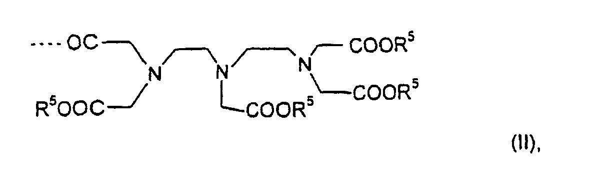 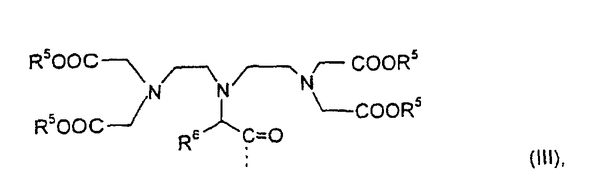 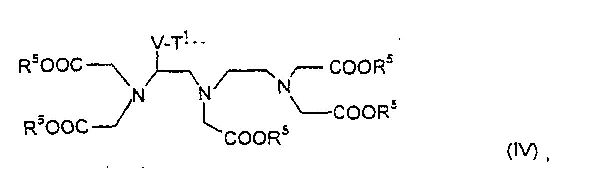 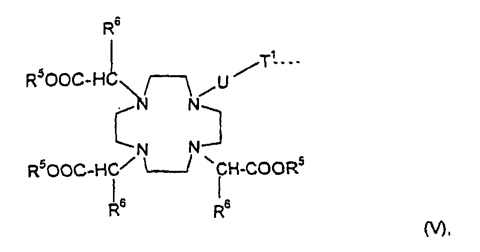 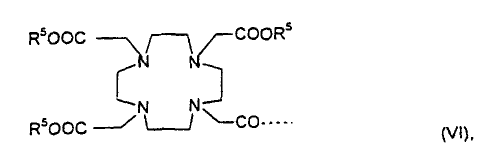 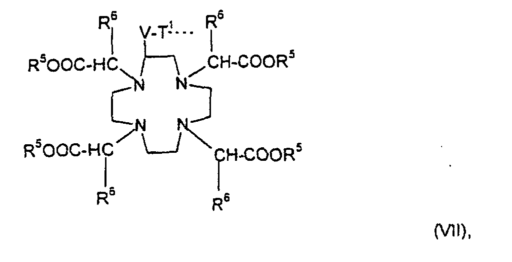 in which
R⁵, independently of one another, mean a hydrogen atom or a metal ion equivalent of the elements of atomic numbers 20-32, 37-39, 42-44, 49 or 57-83,
R⁶ means a hydrogen atom, a straight-chain or branched C₁-C₇ alkyl radical, a phenyl or benzyl radical,
V means a -CH₂-(CH₂)-(O)ₚ 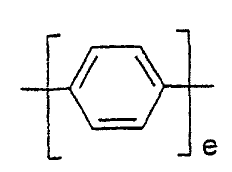 group with
o meaning numbers 0 to 10,
p and e in each case meaning the numbers 0 or 1, provided that p only stands for number 1 if e means number 1,
T¹ means an -NHCS or -CO group,
U means a -CHR⁷- CONR⁷-M¹ or -CH₂-CH(OH)-M² group with R⁷ and R^{7'}, independently of one another, meaning R⁶ or the group -CH₂-(CH₂)ₒ-COOH and M¹ and M² in each case meaning a phenylene radical or a straight-chain, branched, saturated or unsaturated C₁-C₂₀ alkylene chain, which optionally is substituted by 1-5 (CH₂)ₒ-COOH, 1-5 OR⁶ radicals or 1-8 oxygen atoms, 1-2 -NH, 1-2 -C(=NH), 1-5 -CONR⁷, 1-5 -NR⁷CO, 1-2 phenylene or 1-2 phenyleneoxy groups, provided that at least two of radicals R⁵ stand for metal ion equivalents of the elements of the above-mentioned atomic numbers, and optionally cations of inorganic and/or organic bases, amino acids or amino acid amides, 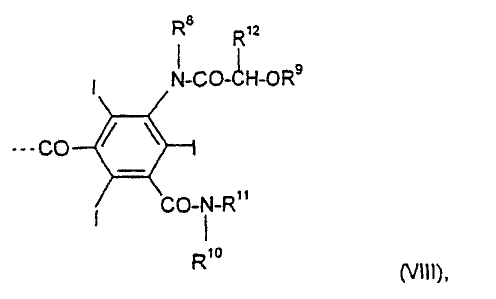 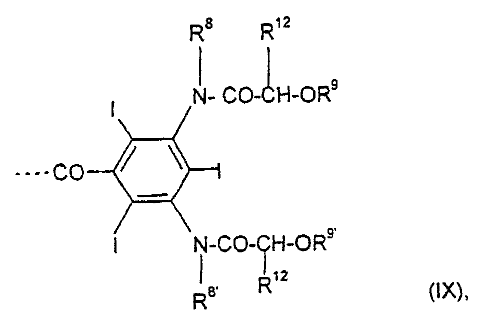 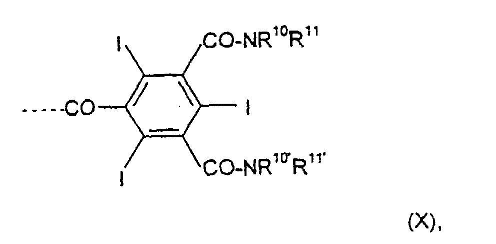 with R⁸, R^{8'}, R¹⁰, R^{10'}, R¹¹, R^{11'}, which can be the same or different, meaning hydrogen or straight-chain alkyl with 2-6 C atoms or branched-chain alkyl with 3-6 C atoms, where both alkyl radicals can be substituted with 1-5 OH groups,
R⁹, R^{9'}, R¹², which can be the same or different, meaning hydrogen or methyl and
Y means the radicals
-W-(CH₂)₂[O-CH₂-CH₂]_{q}-W, 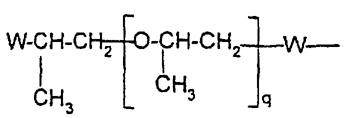
in which q means the numbers 8 to 200, W means the NH group or the O atom, and R³ and R⁴, independently of one another, mean a substituent with a diameter of at least 0.6 nm.

3. Polyrotaxanes of formula I according to claims 2, **characterized in that** the groups that stand for R³ and R⁴ are bonded to Y as ester, amide, ether, thioester, thioamide or carbonate.

4. Polyrotaxanes of formula 1 according to claims 2 and 3, **characterized in that** R³ and R⁴, independently of one another, stand for R¹ or a 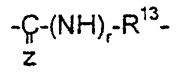 group with Z meaning an oxygen or sulfur atom and r meaning the numbers 0 or 1, where R¹³ stands for a saturated, unsaturated, straight-chain or branched C₁-C₃₀ alkyl chain, which can be interrupted by 1-3 -NH-CO-O, -O-OC-NH, -NHCO, OCNH, NHCS, -SCNH, -NH-CS-NH, -NH-CO-NH, -CO-O, -O-OC, NR⁶, -CO, -SO₂ or -SO groups, 1-2 phenylene, phenyleneoxy or cyclohexylidene groups, or 1-3 oxygen or sulfur atoms, and/or can be substituted by 1-5 -OH, -OCH₃, 1-3 -CH₂OH, -NHCOR⁶, -CONHR⁶, -COOH,-(CH₂)₁₋₅-COOH, -CH₂-C₆H₄-OH, -CH₂-C₆H₅, -C₆H₅, naphthyl, pyridyl, cyclohexyl or thiophenyl groups, where the optionally present aromatic groups optionally can contain 1-3 chlorine, bromine, CH₃, COOH, CH₂OH or OCH₃ substituents.

5. Pharmaceutical agents that contain at least one polyrotaxane complex according to general formula I of claim 2, optionally with the additives that are commonly used in pharmaceutical technology.

6. The use of at least one polyrotaxane complex according to general formula I of claim 2 for the production of agents for NMR or x-ray diagnosis.

7. The use of at least one polyrotaxane complex according to general formula I of claim 2 for the production of agents for angiography.

8. The use of at least one polyrotaxane complex according to general formula I of claim 2 for the production of agents for lymphography.

9. Process for the production of polyrotaxane complexes according to general formula I of claim 2, **characterized in that** compounds of general formula II 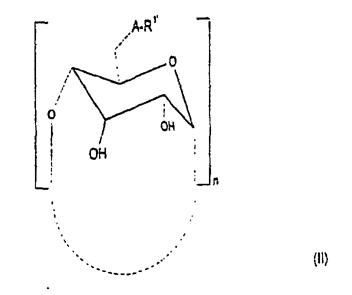 in which A and n have the above-indicated meanings and
R^{1'} stands for a hydrogen atom or the radical II', III', IV', V', VI', VII', VIII', IX' or X', in which the latter have the meaning that is indicated for II-X, but radicals R⁵ that are present in them stand for hydrogen or acid protective groups and optionally present hydroxy groups are optionally present in protected form,
are reacted with compounds of general formula XI
H-Y-H (XI)
to give compounds of general formula XII 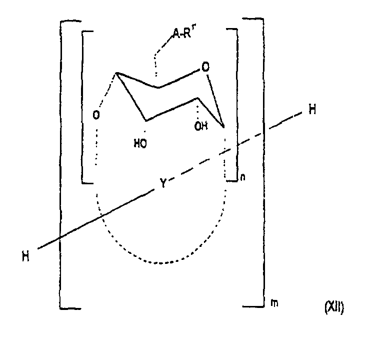 and the latter are reacted with a compound R^{3'}-Fg and/or R^{4'}-Fg, in which R^{3'} and R^{4'} have the meaning that is indicated for R³ and R⁴, but radical R⁵ that is in R¹ stands for hydrogen, acid protective groups or a metal ion equivalent of the elements of the above-mentioned atomic numbers and Fg stands for anhydride, -Cl, -F, -OH, -N₃, 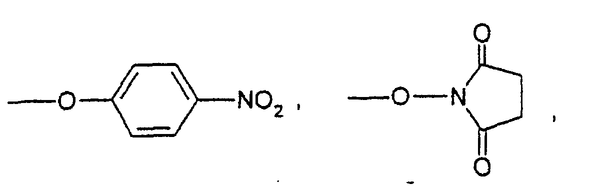 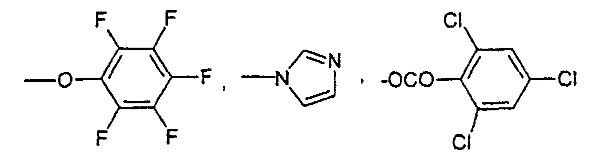 where if R³ and/or R⁴ in (I) stand for a radical 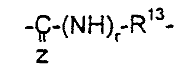 a compound of general formula R³-N=C=Z and/or R⁴-N=C=Z is used, and if T¹ stands for an NHCS group in IV, V and VII, a compound of general formula IV", V" or VII' 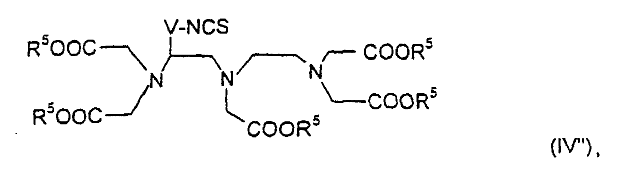 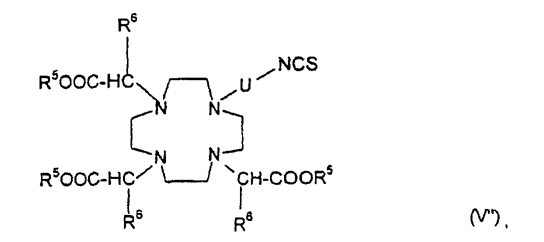 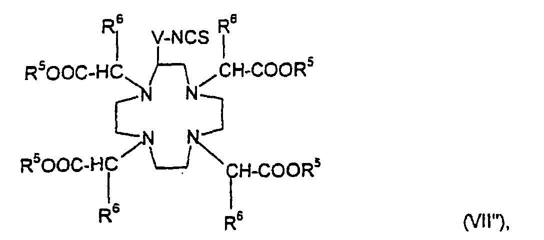 is used, the thus obtained compound of general formula XIII 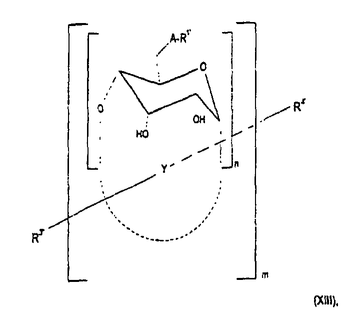 if R^{1'} has the meaning of hydrogen, is reacted with a compound R^{1"}-Fg, in which R^{1"} has the meaning indicated for R¹, but radicals R⁵ that are present in them stand for hydrogen, acid protective groups or a metal ion equivalent of the elements of the above-mentioned atomic numbers, or is reacted with compounds of general formula IV", V" or VII" and then, if R⁵ in XIII does not stand for the above-mentioned metal ion equivalents, optionally present acid protective groups are cleaved off, the desired metal ions are introduced and then, if desired, existing acid hydrogen atoms are substituted by cations of inorganic and/or organic bases, amino acids or amino acid amides.

## Revendications

1. Polyrotaxanes comprenant des complexes métalliques ou des dérivés benzéniques iodés en tant que composants d'imagerie pour le diagnostic par TRM et le diagnostic par rayons X.

2. Polyrotaxanes de formule I 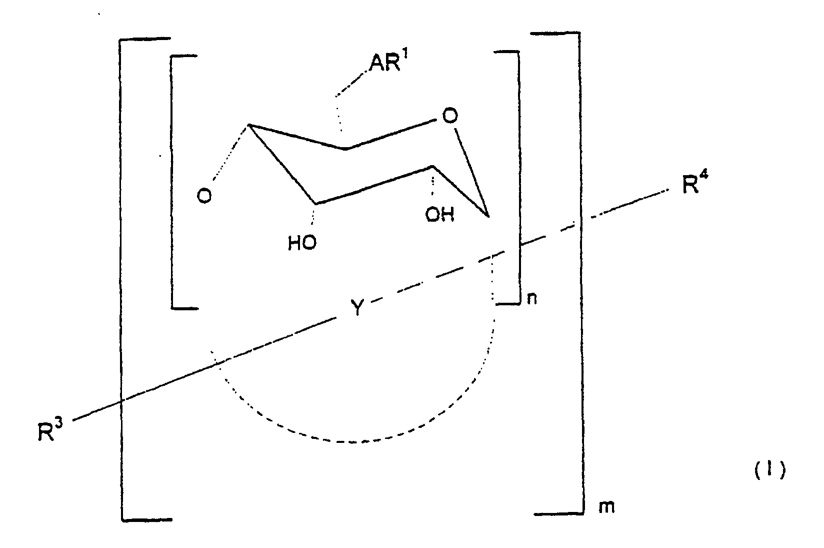 dans laquelle
n représente les nombres 6, 7 ou 8,
m représente les nombres 2 à 50,
A représente un atome d'oxygène ou le groupe -XNH-, où
X représente une liaison directe ou le radical -O-(CO)ₓ-CHR-(CH₂)_{y}-
où x représente les nombres 0 ou 1 et
y représente les nombres 0 à 10,
R¹ représente les radicaux contrastants II, III, IV, V, VI, VII, VIII, IX ou X 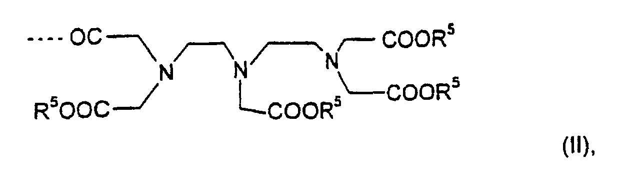 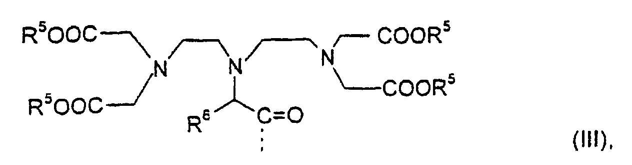 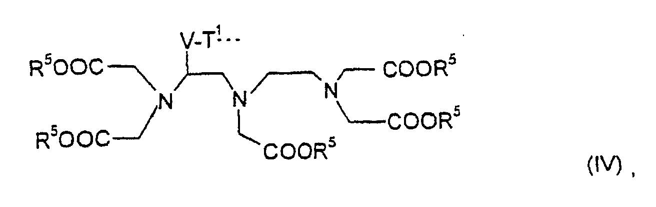 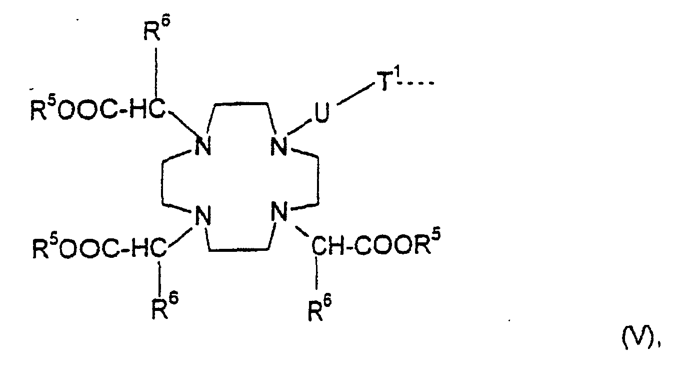 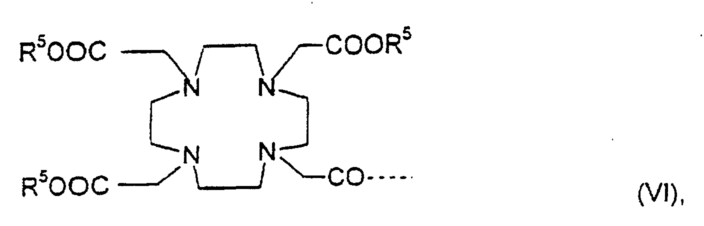 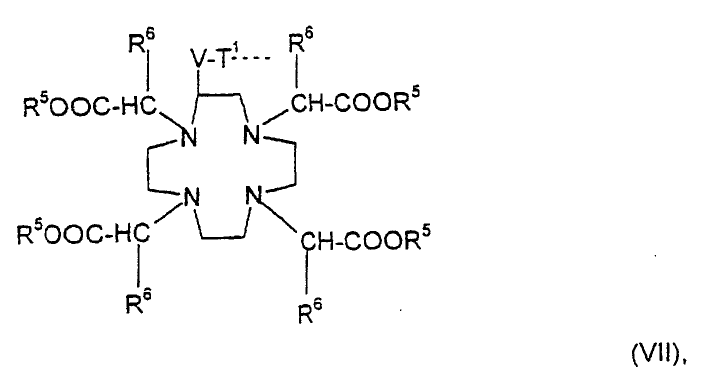 dans lesquels
R⁵ représente indépendamment un atome d'hydrogène ou un équivalent d'ion métallique des éléments de numéros atomiques 20-32, 37-39, 42-44, 49 ou 57-83,
R⁶ représente un atome d'hydrogène ou un radical alkyle en C₁-C₇ linéaire ou ramifié, un radical phényle ou benzyle,
V représente un groupe CH₂-(CH₂)ₒ-(O)ₚ 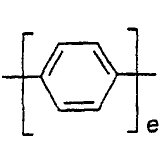 dans lequel
o représente les nombres 0 à 10,
p et e représentent chacun les chiffres 0 ou 1, à condition que p ne représente le chiffre 1 que lorsque e représente le chiffre 1,
T¹ représente un groupe -NHCS ou -CO,
U représente un groupe -CHR⁷-CONR^{7'}-M¹ ou -CH₂-CH(OH) -M² dans lequel R⁷ et R^{7'} représentent, indépendamment l'un de l'autre, R⁶ ou le groupe -CH₂-(CH₂)ₒ-COOH, et M¹ et M² représentent chacun un radical phénylène ou une chaîne alkylène en C₁₋₂₀ linéaire, ramifiée, saturée ou insaturée, qui est éventuellement substituée par 1-5 radicaux (CH₂)ₒ-COOH, 1-5 radicaux OR⁶ ou renferme 1-8 atomes d'oxygène, 1-2 groupes -NH, 1-2 groupes -C(=NH), 1-5 groupes -CONR⁷, 1-5 groupes -NR⁷CO, 1-2 groupes phénylène ou 1-2 groupes phénylène-oxy, à condition qu'au moins deux des radicaux R⁵ représentent des équivalents d'ion métallique des éléments de numéros atomiques susmentionnés, et éventuellement des cations de bases inorganiques et/ou organiques, d'amino-acides ou d'amino-amides, 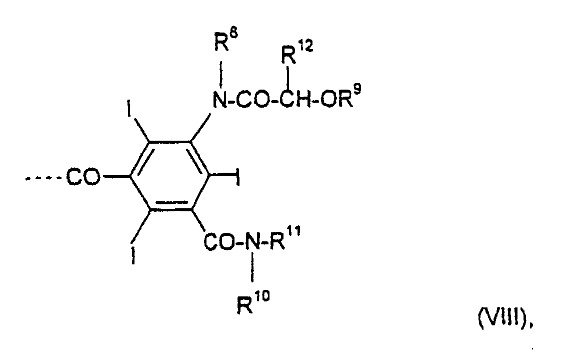 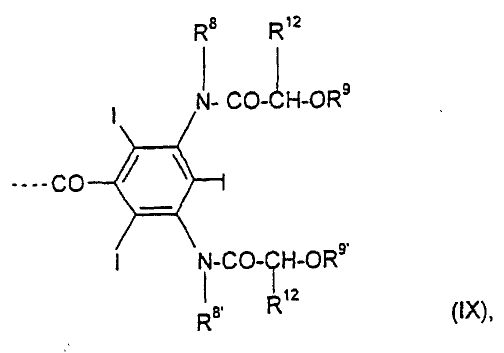 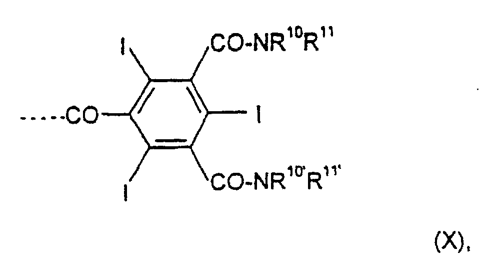 où
R⁸, R^{8'}, R¹⁰, R^{10'}, R¹¹ et R^{11'}, qui peuvent être identiques ou différents, représentent un hydrogène ou un alkyle linéaire ayant de 2 à 6 atomes de carbone ou un alkyle ramifié ayant de 3 à 6 atomes de carbone, les deux radicaux alkyle pouvant être substitués par 1-5 groupes OH,
R⁹, R^{9'} et R¹², qui peuvent être identiques ou différents, représentent un hydrogène ou un méthyle, et Y représente les radicaux -W-(CH₂)₂[O-CH₂-CH₂]_{q}-W, 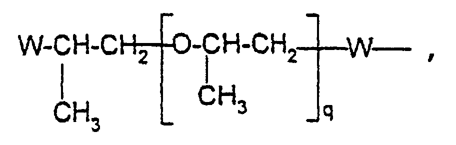 dans lesquels q représente les nombres 8 à 200, W représente le groupe NH ou l'atome d'oxygène, et
R³ et R⁴ représentent, indépendamment l'un de l'autre, un substituant présentant un diamètre d'au moins 0,6 nm.

3. Polyrotaxanes de formule I selon la revendication 2, **caractérisés en ce que** les groupes représentant R³ et R⁴ sont liés à Y sous forme d'ester, d'amide, d'éther, de thioester, de thioamide ou de carbonate.

4. Polyrotaxanes de formule I selon les revendications 2 et 3, **caractérisés en ce que** R³ et R⁴ représentent, indépendamment l'un de l'autres, R¹ ou un groupe 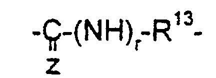 dans lequel Z représente un atome d'oxygène ou de soufre et r représente les nombres 0 ou 1, où R¹³ représente une chaîne alkyle en C₁-C₃₀ saturée, insaturée, linéaire ou ramifiée, qui peut être interrompue par 1-3 groupes -NH-CO-O-, -O-OC-NH-, -NHCO-, OCNH-, NHCS-, -SCNH-, -NH-CS-NH-, -NH-CO-NH-, -CO-O-, -O-OC-, NR⁶-, -CO-, -SO₂-, -SO-, 1-2 groupes phénylène, phénylène-oxy, cyclohexylidène, 1-3 atomes d'oxygène, de soufre et/ou peut être substituée par 1-5 groupes -OH-, -OCH₃-, 1-3 groupes -CH₂OH-, -NHCOR⁶-, -CONHR⁶-, -COOH-, -(CH₂)₁₋₅-COOH-, -CH₂-C₆H₄OH-, -CH₂-C₆H₅-, -C₆H₅-, naphtyle, pyridyle, cyclohexyle, triophényle, les groupes aromatiques éventuellement présents pouvant éventuellement renfermer 1-3 substituants chloro, bromo, CH₃, COOH, CH₂OH, OCH₃.

5. Compositions pharmaceutiques comprenant au moins un complexe de polyrotaxane selon la formule générale I de la revendication 2, éventuellement avec les additifs habituels en galénique.

6. Utilisation d'au moins un complexe de polyrotaxane selon la formule générale I de la revendication 2 pour la préparation de compositions destinées au diagnostic par RMN ou au diagnostic par rayons X.

7. Utilisation d'au moins un complexe de polyrotaxane selon la formule générale I de la revendication 2 pour la préparation de compositions destinées à l'angiographie.

8. Utilisation d'au moins un complexe de polyrotaxane selon la formule générale I de la revendication 2 pour la préparation de compositions destinées à la lymphographie.

9. Procédé de préparation de complexes de polyrotaxane selon la formule générale I de la revendication 2, **caractérisé en ce que** des composés de formule générale II 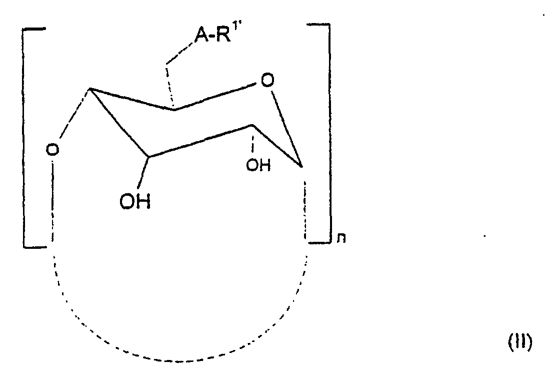 dans laquelle
A et n présentent les significations indiquées ci-dessus et
R^{1'} représente un atome d'hydrogène ou le radical II', III', IV', V', VI', VII', VIII', IX' ou X', où ceux-ci présentent la signification indiquée pour II - X, mais les radicaux R⁵ qui y sont présents représentent un hydrogène ou des groupes protecteurs d'acides et les groupes hydroxy éventuellement présents se trouvent éventuellement sous forme protégée,
sont mis à réagir avec des composés de formule générale XI
H-Y-H (XI)
pour donner lieu à des composés de formule générale XII 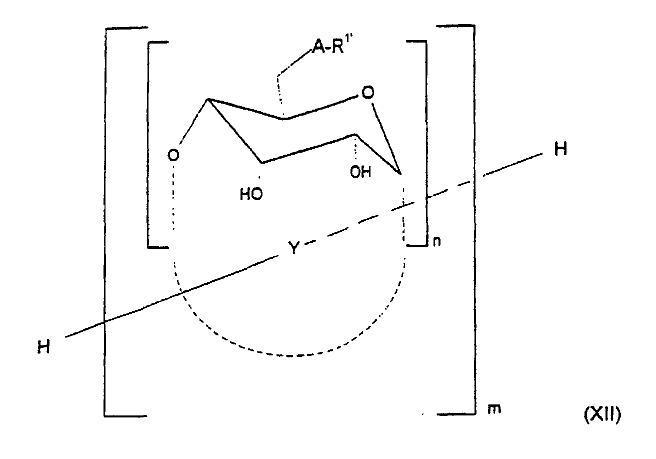 et ceux-ci sont mis à réagir avec un composé R^{3'}-Fg et/ou R^{4'}-Fg,
où R^{3'} et R^{4'} présentent la signification indiquée pour R³ et R⁴, mais les radicaux R⁵ présents dans R¹ représentent un hydrogène, des groupes protecteurs d'acides ou un équivalent d'ion métallique des éléments de numéros atomiques mentionnés, et Fg représente un anhydride, -Cl, -F, -OH, -N₃, 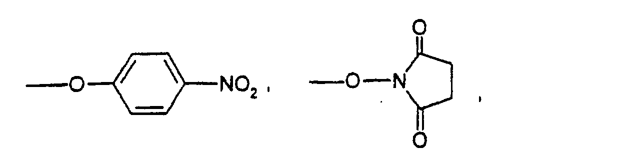 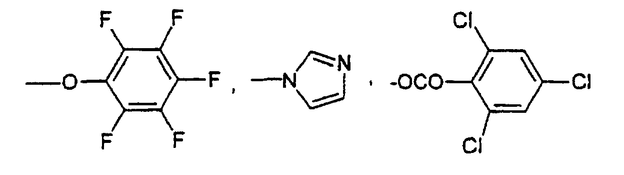 où
dans le cas où R³ et/ou R⁴ dans (I) représentent un radical 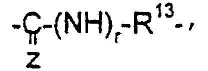 on utilise un composé de formule générale R³-N=C=Z ou R⁴-N=C=Z, et dans le cas où T¹ représente un groupe NHCS en IV, V et VII, on utilise un composé de formule générale IV", V" ou VII" 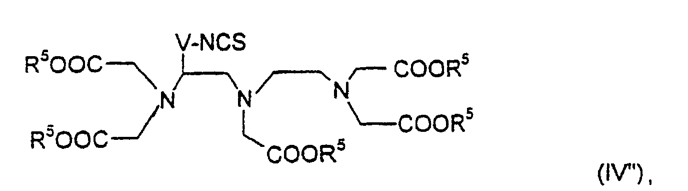 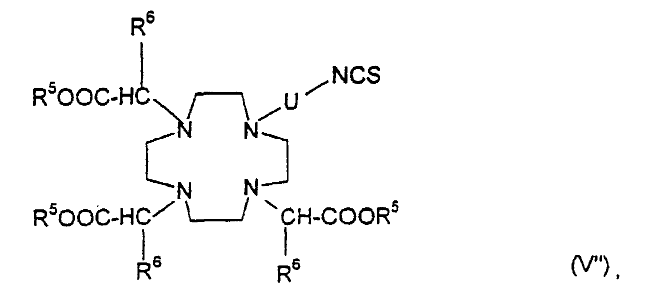 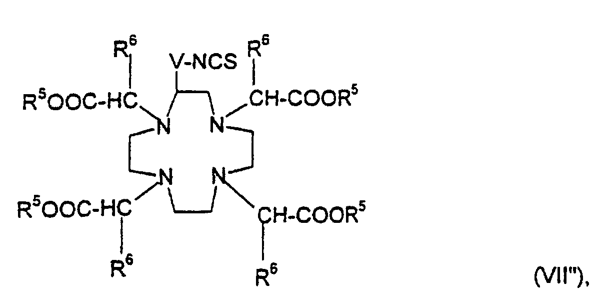 le composé ainsi obtenu de formule générale XIII 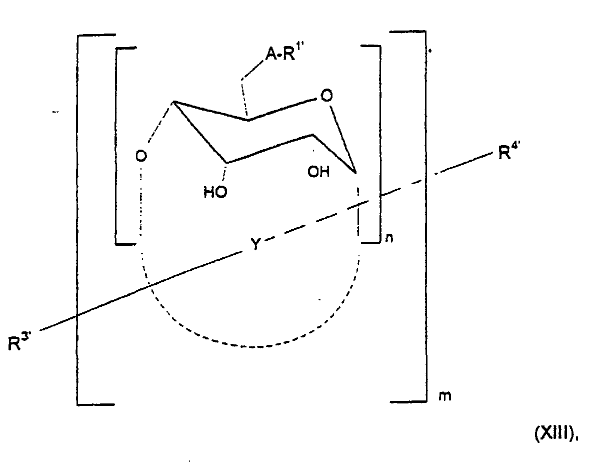 est mis à réagir, si R^{1'} représente un hydrogène, avec un composé R^{1"}-Fg, dans lequel R^{1"} présente la signification indiquée pour R¹, mais les radicaux R⁵ qui y sont présents représentent un hydrogène, des groupes protecteurs d'acides ou un équivalent d'ion métallique des éléments de numéros atomiques mentionnés, ou avec des composés de formule générale IV", V" ou VII", et ensuite, au cas où R⁵ dans XIII ne représente pas les équivalents d'ion métallique susmentionnés, les groupes protecteurs d'acides éventuellement présents sont éliminés, les ions métalliques souhaités sont introduits et ensuite, si on le souhaite, les atomes d'hydrogène acides présents sont substitués par des cations de bases inorganiques et/ou organiques, d'amino-acides ou d'amino-amides.
